# EUROPEAN PATENT APPLICATION

(11) **EP 1 862 454 A1**
(43) Date of publication of application: **05.12.2007**
(21) Application number: 06011490.7
(22) Date of filing: 02.06.2006
(51) Int. Cl.: C07C 335/12, A61K 31/18, A61P 29/00

(54) **Novel compounds, isomer thereof or pharmaceutically acceptable salts thereof as vanilloid receptor antagonist; and a pharmaceutical composition containing the same**

(71) Applicant: Amorepacific Corporation, Seoul 140-777 (KR)
(72) Inventor: Kim, Hee-Doo, College of Pharmacy, Youngsan-Ku, Seoul 140-742 (KR); Suh, Young-Ger, College of Pharmacy, Gwanak-gu, Seoul 151-010 (KR); Park, Hyeung-Geun , College of Pharmacy, Gwanak-gu, Seoul 151-010 (KR); Oh, Uh Taek , College of Pharmacy, Gwanak-gu, Seoul 151-010 (KR); Park, Young -Ho, Dongjak-ku, Seoul 156-775 (KR); Shin, Song Seok, Yongin-si,Gyeonggi-do 449-959 (KR); Lee, Ki-Wha, Gangnam-gu,Seoul 135-230 (KR); Kim, Sun-Young, Seocho-gu, Seoul 137-873 (KR); Kim, Jin Kwan, Paldal-ku,Suwon-si,Kyunggi-do 442-831 (KR); Jeong, Yeon Su, Yongin-si, Kyunggi-do 449-749 (KR); Woo, Byoung Young, Cheoin-gu,Yongin-si, Gyeonggi-do 449-722 (KR)
(74) Representative: Blodig, Wolfgang

(57) **Abstract**

This present invention relates to compounds of formula (I), in which R₃ represents C2-C5 alkenyl or C2-C5 alkynyl and the other variables are as defined in the claims, isomers thereof or pharmaceutically acceptable salts thereof as vanilloid receptor (Vanilloid Receptor 1; VR1; TRPV1) antagonists; and a pharmaceutical composition containing the same.
The present invention provides a pharmaceutical composition for preventing or treating pain, inflammatory disease of the joints, urinary bladder hypersensitivity including urinary incontinence, stomach duodenal ulcer, irritable bowel syndrome, inflammatory bowel disease, neurotic/allergic/inflammatory skin disease, psoriasis, asthma, chronic obstructive pulmonary disease, pruritus or prurigo.

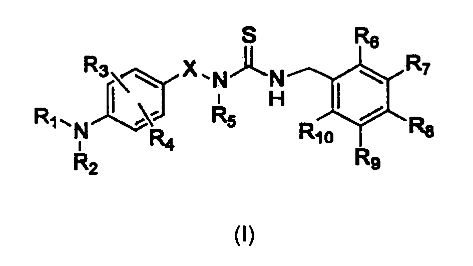

## Description

### Technical field

The present invention relates to novel compounds, isomer thereof or pharmaceutically acceptable salts thereof as vanilloid receptor antagonist; and a pharmaceutical composition containing the same

### Background Art

As diseases associated with the activity of vanilloid receptor (Nagy et al., 2004, Eur. J. Pharmacol. 500, 351-369) pain such as acute pain, chronic pain, neuropathic pain, post-operative pain, rheumatic arthritic pain, osteoarthritic pain, postherpetic neuralgia, neuralgia, headache, and migraine(Petersen et al., 2000, Pain, 88, pp125-133; Walker et al., 2003, J. Pharmacol. Exp. Ther., 304, pp56-62); nerve-related diseases such as neuropathies, HIV-related neuropathy, nerve injury, neurodegeneration, and stroke(Park et al., 1999, Arch. Pharm. Res. 22, pp432-434; Kim et al., 2005, J. Neurosci. 25(3), pp662-671); diabetic neuropathy(Kamei et al., 2001, Eur. J. Pharmacol. 422, pp83-86); fecal urgency; irritable bowel syndrome (Chan et al., 2003, Lancet, 361, pp385-391); inflammatory bowel disease (Yiangou et al., 2001, Lancet, 357, pp1338-1339); disease of digestive organ such as stomach-duodenal ulcer and Crohn's disease(Holzer P, 2004, Eur. J. Pharm. 500, pp231-241; Geppetti et al., 2004, Br. J. Pharmacol., 141, pp1313-1320); respiratory diseases such as asthma, chronic obstructive pulmonary disease(Hwang et al., 2002, Curr. Opin. Pharmacol. pp235-242; Spina et al., 2002, Curr. Opin. Pharmacol. pp264-272); urinary incontinence (Birder et al., 2002, Nat. Neuroscience, 5, pp856-860); urinary bladder hypersensitiveness (Birder et al., 2001, Proc. Natl. Acad. Sci. 98, pp13396-13401); neurotic/allergic/inflammatory skin disease such as psoriasis, pruritus and prurigo(Southall et al., 2003, J. Pharmacol. Exp. Ther., 304, pp217-222); irritation of skin, eye or mucous membrane (Tominaga et al., 1998, Neuron 21 pp531-543); hyperacusis; tinnitus; vestibular hypersensitiveness (Balaban et al., 2003, Hear Res. 175, pp165-70); cardiac disease such as myocardial ischemia etc.(Scotland et al., 2004, Circ. Res. 95, pp1027-1034; Pan et al., 2004, Circulation, 110, pp1826-1831) can be enumerated.

The vanilloid receptor (VR1) is the receptor for capsaicin (8-methyl-N-vanillyl-6-nonenamide), a pungent ingredient in hot peppers. The molecular cloning thereof was also reported in 1997 (Caterina et al., 1997, Nature 389, pp816-824). This receptor is a non-selective cation channel composed of 6 transmembrane domains and belongs to the TRP channel family. Recently, it was named TRPV1. On the other hand, it is known that the vanilloid receptor is activated by stimuli such as capsaicin, resiniferatoxin, heat, acids, anandamide, lipid metabolites or the like; thus it plays a crucial role as a molecular integrator of physico-chemically noxious stimuli in mammals (Tominaga et al., 1998, Neuron 21 pp531-543; Hwang et al., 2000, PNAS, 97, pp6155-6160). Activation of the vanilloid receptor by endogenous/exogenous stimuli leads to not only transmission of noxious stimuli, but also liberation of neuropeptides such as substance P, CGRP (Calcitonin Gene-Related Peptide) and the like, thereby causing neurogenic inflammation. The vanilloid receptor is highly expressed in primary afferent sensory neurons. It is also reportedly expressed in various organs and tissues such as the bladder, kidney, lung, intestine and skin, and in the central nervous system (CNS) including the brain and non-neuronal tissues (Mezey et al., 2000, PNAS, 97, pp3655-3660; Stander et al., 2004, Exp. Dermatol. 13, pp129-139; Cortright et al., 2001, BBRC, 281, pp1183-1189). In particular, TRPV1 receptor knock-out mice exhibit a normal response to harmful physical stimuli, but show a reduced response to painful heat and vanilloids, and exhibit little hyperalgesia to thermal stimuli even in an inflammatory state (Caterina et al., 2000, Science 288, pp306-313; Davis et al., 2000, Nature 405, pp183-187; Karai et al., 2004, J. Clin. Invest., 113, pp1344-1352). Lately, an additional role of the vanilloid receptor is also expected by the report of possibility that the vanilloid receptor may be present in the form of a heteromultimer with TRPV3, another TRP channel (Smith et al., 2002, Nature, 418, pp186-190).

As mentioned above, the vanilloid receptor knock-out mice exhibited reduced responses to thermal or noxious stimuli, thus raising the possibility that vanilloid receptor antagonists may be utilized for prevention or treatment of various pain conditions. Recently, this possibility is supported by the report that the well-known vanilloid receptor antagonist, capsazepine also decreases hyperalgesia caused by physical stimuli in models of inflammatory and neuropathic pain (Walker et al., 2003, JPET, 304, pp56-62; Garcia-Martinez et al., 2002, Proc. Natl. Acad. Sci. 99, 2374-2379). In addition, treatment of the primary culture of afferent sensory neurons with the vanilloid receptor agonist, capsaicin etc., results in damage to nerve functions and furthermore death of nerve cells. The vanilloid receptor antagonist exerts defense actions against such damage to nerve functions and nerve cell death (Holzer P, 1991, Pharmacological Reviews, 43, pp143-201; Mezey et al., 2000, PNAS, 97, 3655-3660). The vanilloid receptor is expressed on sensory neurons distributed in myenteric ganglia, muscle layer, mucosa and epithelial cells in all regions of the gastrointestinal tract. In particular, the vanilloid receptor is highly expressed in inflammatory disorders of the colon and ileum.

In addition, activation of the vanilloid receptor stimulates sensory nerves, which in turn causes release of neuropeptides which are known to play a critical role in pathogenesis of bowel disorders. The role of the vanilloid receptor in development of gastrointestinal disorders is well elucidated and documented in recent scientific papers and journals, for example, Holzer P, 2004, Eur. J. Pharm. 500, pp231-241; Geppetti et al., 2004, Br. J. Pharmacol., 141, pp1313-1320. According to such references, it seems that the vanilloid receptor antagonists will be effective for prevention or treatment of gastrointestinal diseases such as gastro-esophageal reflux disease (GERD) and gastroduodenal ulcer (DU). It has been reported that the number of sensory nerves expressing the vanilloid receptor is increased in patients suffering from irritable bowel syndromes and such increased expression of the vanilloid receptor is known to be involved in the development of the disease (Chan et al., 2003, Lancet, 361, pp385-391). Other investigations showed that expression of the vanilloid receptor is significantly increased in patients suffering from inflammatory bowel disorders. Taken together, it appears that the vanilloid receptor antagonist may also be therapeutically effective for such bowel disorders (Yiangou et al., 2001, Lancet, 357, pp1338-1339). The vanilloid receptor-expressing afferent nerves are abundantly distributed in airway mucosa. Bronchial hypersensitivity is very similar to hyperalgesia, and protons and lipoxygenase products, known as endogenous ligands for the vanilloid receptor, are well known as crucial factors responsible for development of asthma and chronic obstructive pulmonary diseases (Hwang et al., 2002, Curr. Opin. Pharmacol. pp235-242; Spina et al., 2002, Curr. Opin. Pharmacol. pp264-272). Further, it has been reported that air-polluting substances, which are a kind of asthma-causing substances, i.e., particulate matter specifically acts on the vanilloid receptor and such action is inhibited by capsazepine, thus suggesting the possible applicability of vanilloid receptor antagonists to respiratory diseases (Veronesi et al., 2001, NeuroToxicology, 22, pp795-810). Urinary bladder hypersensitiveness and urinary incontinence are caused by various central/peripheral nerve disorders or injury, and capsaicin-responsive sensory nerves play an important role in bladder function control and inflammation. In addition, immunoreactivity of the vanilloid receptor was reported in urinary bladder epithelium (urothelium) in rats and it was found that bladder overactivity induced by capsaicin was due to stimulation of vanilloid receptors present in nerve fibers, or various transmitters which are released by vanilloid receptors (Birder et al., 2001, Proc. Natl. Acad. Sci. 98, pp13396-13401). Further, VR1 (TRPV1) -/- mice are anatomically normal, but exhibit higher frequency of low-amplitude, non-voiding bladder contractions, as compared to wild type mice, thus indicating that the vanilloid receptor affects functions of the bladder (Birder et al., 2002, Nat. Neuroscience, 5, pp856-860). Some of vanilloid agonists are recently under development as therapeutics for treating bladder diseases. Vanilloid receptors are distributed in human epidermal keratinocytes as well as in primary afferent sensory nerves(Denda et al., 2001, Biochem. Biophys. Res. Commun., 285, pp1250-1252; Inoue et al., 2002, Biochem. Biophys. Res. Commun., 291, pp124-129), and are then involved in transmission of various noxious stimuli and pains such as skin irritation and pruritus, thereby having close correlation with etiology of dermatological diseases and disorders such as skin inflammation, due to neurogenic/non-neurogenic factors. This is supported by the report that the vanilloid receptor antagonist, capsazepine inhibits inflammatory factors in human skin cells (Southall et al., 2003, J. Pharmacol. Exp. Ther., 304, pp217-222).

Based on the above-mentioned information, development of various vanilloid receptor antagonists is under way, and some patents and patent applications relating to vanilloid receptor antagonists under development were recently published, in which the above mentioned information is described well (Rami et al., 2004, Drug Discovery Today: Therapeutic Strategies, 1, pp97-104).

As a result of extensive and intensive studies based on the theoretical background discussed above, the present inventors have synthesized novel compounds having antagonistic activity by selective action on a vanilloid receptor and thus completed the present invention.

Consequently, present inventors have found, surprisingly, that by substituting the C2-C5 alkenyl or C2-C5 alkynyl group in their phenyl ring carrying an amine-containing substituent, the compounds are obtained that possess very potent vanilloid receptor antagonistic activity.

Therefore, it is an object of the present invention to provide novel compounds useful as a potent antagonist for a vanilloid receptor, isomer thereof and pharmaceutically acceptable salts thereof; and a pharmaceutical composition comprising the same.

### Disclosure of the invention

The present invention provides a novel compound of the following formula (I), an isomer thereof, or pharmaceutically acceptable salts thereof; a pharmaceutical composition containing the same 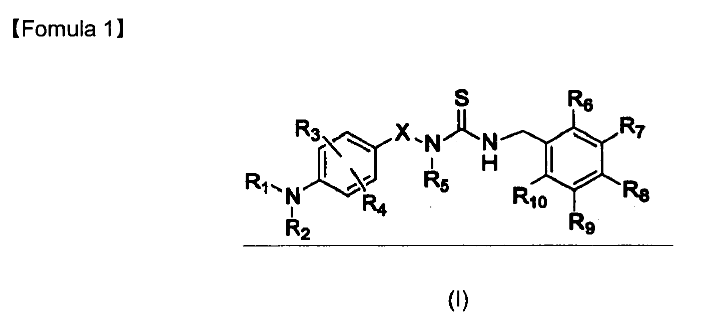 wherein,
X represents CHR₁₁ wherein R₁₁ represents hydrogen, halogen, or C1-C5 alkyl;
R₁ and R₂ independently represent hydrogen, -SO₂R₁₂, -SOR₁₂, C1-C5 alkyl, C1-C5 alkoxy, halo (C1-C5) alkyl, C2-C5 alkenyl, phenyl, phenyl (C1-C3) alkyl, or C1-C3 alkoxyphenyl, wherein R₁₂ represents hydrogen, C1-C5 alkyl, C2-C5 alkenyl, trifluoromethyl, phenyl, or benzyl;
R₃ represents C2-C5 alkenyl or C2-C5 alkynyl;
R₄ represents hydrogen, carboxy, C1-C5 alkyl, halogen, nitro, cyano, C2-C5 alkenyl, C2-C5 alkynyl, C1-C5 alkoxy, halo (C1-C5) alkyl, C1-C5 alkylcarbonyl, C1-C5 alkylcarbonylamino, C1-C5 alkylsulfonylamino, phenylsulfonylamino, or C1-C5 alkoxycarbonyl;
R₅ represents hydrogen, hydroxy, C1-C5 alkyl, or halo (C1-C5) alkyl; and
R₆, R₇, R₈, R₉ and R₁₀ independently represent hydrogen, C1-C5 alkyl, C2-C5 alkenyl, C2-C5 alkynyl, C1-C5 alkoxy, halo (C1-C5) alkyl, C1-C5 alkylthio, C1-C5 alkylsulfonyl, or halogen.

One preferred aspect of the present invention is a compound of the formula (I), an isomer thereof, or a pharmaceutically acceptable salt thereof,
wherein,
X represents CHR₁₁ wherein R₁₁ represents hydrogen, halogen, or C1-C5 alkyl;
R₁ and R₂ independently represent hydrogen, -SO₂R₁₂, -SOR₁₂, C1-C5 alkyl, C1-C5 alkoxy, halo (C1-C5) alkyl, or C2-C5 alkenyl, wherein R₁₂ represents hydrogen, C1-C5 alkyl, C2-C5 alkenyl, or trifluoromethyl;
R₃ represents C2-C5 alkenyl or C2-C5 alkynyl;
R₄ represents hydrogen, C1-C5 alkyl, halogen, C2-C5 alkenyl, C2-C5 alkynyl, or halo (C1-C5) alkyl;
R₅ represents hydrogen, hydroxy, C1-C5 alkyl, or halo (C1-C5) alkyl; and
R₆, R₇, R₈, R₉ and R₁₀ independently represent hydrogen, C1-C5 alkyl, C2-C5 alkenyl, C2-C5 alkynyl, C1-C5 alkoxy, halo (C1-C5) alkyl, C1-C5 alkylthio, C1-C5 alkylsulfonyl, or halogen.

In the above, compounds of formula (I) are preferred in which R₃ represents ethenyl, ethynyl, propenyl, or propynyl; and R₄ represents hydrogen, halogen, methyl, ethyl, propyl, isopropyl, butyl, isobutyl, ethenyl, ethynyl, or trifluoromethyl.

In the above, compounds of formula (I) are also preferred in which R₁ represents hydrogen, and R₂ represents -SO₂R₁₂, wherein R₁₂ represents C1-C3 alkyl; and R₈ represents isopropyl, trifluoromethyl or tert-butyl.

More preferably, in the above formula (I),
X represents CHR₁₁ wherein R₁₁ represents hydrogen or C1-C5 alkyl;
R₁ represents hydrogen, and R₂ represents -SO₂R₁₂, wherein R₁₂ represents C1-C3 alkyl;
R₃ represents ethenyl or ethynyl;
R₄ represents hydrogen, halogen, methyl, ethyl, propyl, isopropyl, butyl, isobutyl, or trifluoromethyl;
R₅, R₆, R₇, R₉ and R₁₀ represent hydrogen; and
R₈ represents tert-butyl.

Even more preferred aspect of the present invention is a compound according to the formula (1a), an isomer, or a pharmaceutically acceptable salt thereof; 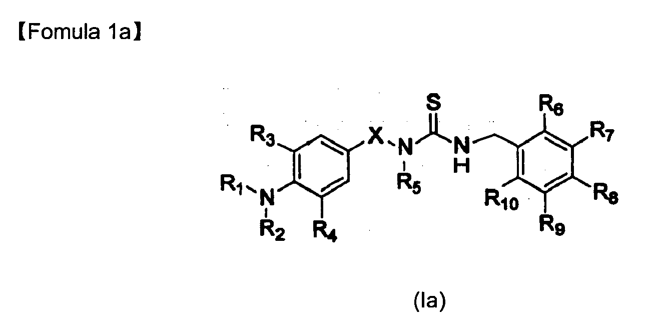 wherein,
R₁ represents hydrogen, and R₂ represents methanesulfonyl; and
R₃ and R₄ are both bounded to the phenyl ring in ortho-position to the methanesulfonylamino group.

Preferred examples of compounds according to the invention are selected from the group consisting of,
N-{4-[3-(4-t-butylbenzyl)thioureido-methyl]-2-vinyl-phenyl}methanesulfonamide,
N-{4-[3-(4-t-butylbenzyl)thioureido-methyl]-2-trifluoromethyl-6-vinyl-phenyl}methanesulfonamide,
N-{4-[3-(4-t-butylbenzyl)thioureido-methyl]-2-fluoro-6-vinyl-phenyl}methanesulfonamide,
N-{4-[3-(4-t-butylbenzyl)thioureido-methyl]-2-chloro-6-vinylphenyl}methanesulfonamide,
N-{4-[3-(4-t-butylbenzyl)thioureido-methyl]-2-methyl-6-vinylphenyl}methanesulfonamide, and
(R)-N-(4-{1-[3-(4-t-butylbenzyl)thioureido]ethyl}-2-fluoro-6-vinyl-phenyl)methanesulfonamide.

The compounds of the formula (I) of the present invention can chemically be synthesized by the following reaction schemes. However, these are given only for illustration of the invention and not intended to limit them.

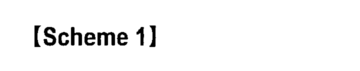

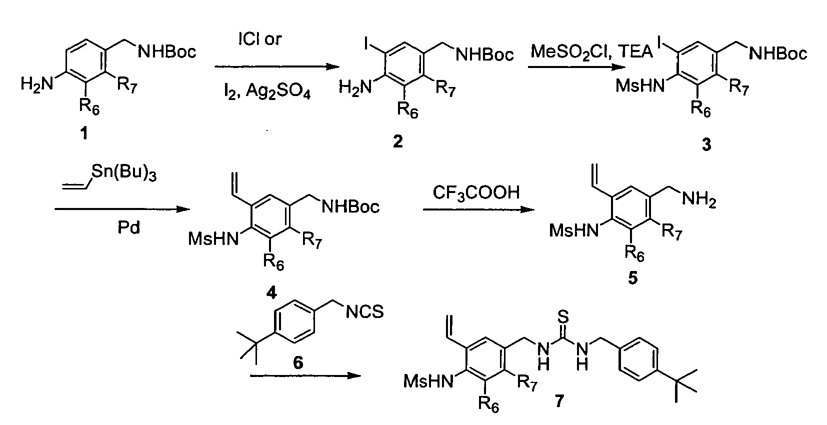

The Scheme 1 shows the five-step reaction to prepare thiourea with vinyl moiety. In first step, the iodo group is introduced into the neighboring location of amino group of the compound (1) to yield the compound (2). Iodination of the compound can be achieved by using iodine and silver sulfate or ICI. The methanesulfonyl group is introduced to the compound (2) to yield the compound (3). Vinyl stannane is coupled with iodo group of the compound (3) to yield the compound (4). The protecting group (Boc) is removed by using TFA (CF₃COOH) to yield the benzyl amine compound (5). The benzyl amine compound (5) is reacted with 4-t-butylbenzylthioisocyanate (6) to yield the thiourea compound (7) with vinyl group.

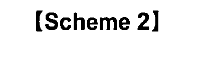

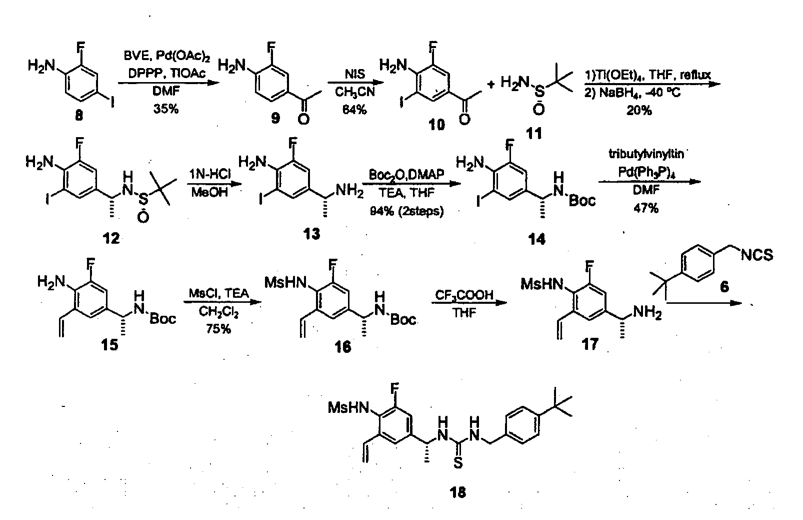

The Scheme 2 shows a process for synthesizing the stereospecific thiourea compound (18). Butylvinylether is coupled to the iodoaniline compound (8) to yield the compound (9). Iodination of the compound (9) can be achieved by using NIS. The iodoaniline compound (10) is reacted with (R)-(+)-2-methyl-2-propane-2-sulfinamide (11) to yield 2-methylpropane-2-sulfinic acid [1-(4-amino-3-fluoro-5-iodophenyl)ethyl]amide (12). The t-butylsulfinyl moiety of the compound (12) is eliminated with 1 N HCl solution to yield the compound (13). The aminomethyl group of the compound (13) was protected with Boc₂O to yield compound (14). Vinylstannane is coupled with iodo group of the compound (14) to yield the compound (15). The methanesulfonyl group is introduced into the amino group to prepare the compound (16). The stereospecific thiourea compound (18) is prepared according to similar procedure in the Scheme 1.

The present invention also provides a pharmaceutical composition comprising a compound of formula (l), an isomer thereof, or a pharmaceutically acceptable salt thereof as an active ingredient together with a pharmaceutically acceptable carrier.

In said pharmaceutical composition, a compound of formula (l), an isomer thereof, or a pharmaceutically acceptable salt thereof as an active ingredient together with an pharmaceutically acceptable carrier is present in an effective amount for preventing or treating pain, acute pain, neuropathic pain, post-operative pain, migraine, arthralgia, neuropathies, nerve injury, diabetic neuropathy, neurodegeneration, stroke, neurotic/allergic/inflammatory skin disease, psoriasis, pruritus, prurigo, urinary bladder hypersensitiveness, irritable bowel syndrome, fecal urgency, Crohn's disease, respiratory disorder such as asthma or chronic obstructive pulmonary disease, irritation of skin, eye or mucous membrane, stomach-duodenal ulcer, inflammatory bowel disease or inflammatory diseases.

The present invention also provides a pharmaceutical composition for preventing and treating a disease associated with the pathological stimulation and/or aberrant expression of vanilloid receptor, wherein said composition comprises a compound of formula (l), an isomer thereof or a pharmaceutically acceptable salt thereof; and pharmaceutically acceptable carrier.

The present invention also provides a pharmaceutical composition for preventing and treating a condition related to vanilloid receptor, where said composition comprises a compound of formula (l), an isomer thereof or a pharmaceutically acceptable salt thereof and pharmaceutically acceptable carrier.

In the above, said condition related to vanilloid receptor is pain, migraine, arthralgia, neuralgia, neuropathies, nerve injury, skin disorder, urinary bladder hypersensitiveness, irritable bowel syndrome, fecal urgency, a respiratory disorder, irritation of skin, eye or mucous membrane, stomach-duodenal ulcer, inflammatory diseases, ear disease, or heart disease.

More specifically, said condition related to vanilloid receptor is acute pain, chronic pain, neuropathic pain, post-operative pain, rheumatic arthrodynia, osteoarthritis pain, postherpetic neuralgia, diabetic neuropathy, HIV-related neuropathy, neurodegeneration, stroke, neurotic/allergic/inflammatory skin disease, psoriasis, pruritus, vitiligo, prurigo, asthma, chronic obstructive pulmonary disease, urinary incontinence, inflammatory bowel disease, hyperacusis, tinnitus, vestibular hypersensitiveness, or inotropic ischemia.

In one preferred aspect, the present invention provides a pharmaceutical composition for treating a condition selected from pain, inflammatory disease of the joints including autoimmune diseases of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease (COPD), pruritus or prurigo comprising a compound, an isomer thereof or a pharmaceutically acceptable salt thereof according to formula (l) as defined further above.

More specifically, the inventive compounds can be used in a pharmaceutical composition for treating pain, wherein the pain is -or is associated with- a condition selected from osteoarthritis ("OA"), rheumatoid arthritis ("RA"), Ankylosing Spondylitis ("AS"), diabetic neuropathic pain, post-operative pain, non-inflammatory musculoskeletal pain (including fibromyalgia, myofascial pain syndrome and back pain), migraine and other types of headaches.

If the compounds of the present invention are said to be useful to treat pain associated with osteoarthritis, it shall not be excluded that this also comprises the treatment of other signs and symptoms of osteoarthritis. Besides reducing the pain associated with osteoarthritis, the pharmacological intervention of osteoarthritis may be aimed at maintaining the mobility and minimizing the disability of the joints.

The term "osteoarthritis (OA)" as used herein typically includes diseases with a failure of a diarthrodial (movable, synovial-lined) joint. In idiopathic (primary) OA, the most common form of the disease, no predisposing factor is apparent. Secondary OA is attributable to an underlying cause. Pain and joint dysfunction are the cardinal symptoms of OA. The joint pain of OA is often described as a deep ache and is localized to the involved joint. Typically, the pain of OA is aggravated by joint use and relieved by rest, but, as the disease progresses, it may become persistent. Nocturnal pain, interfering with sleep, is seen particularly in advance OA of the hip and may be enervating. Stiffness of the involved joint on arising in the morning or after a period of inactivity may be prominent but usually lasts less than 20 minutes.

If the compounds according to the present invention are said to be of use in treating pain associated with an inflammatory autoimmune disease of the joints, this refers to the administration of the compounds or combinations of the compounds of the present invention to reduce at least one pain symptom experienced by a subject suffering from an inflammatory autoimmune disease of the joints including back pain, joint pain and muscle pain associated with RA or AS. Besides the pain relief, treatment of an inflammatory autoimmune disease of the joints may also include a decrease of the inflammation and/or swelling of the synovium and may help to improve the functionality (i.e. maintaining mobility, and minimizing disability) of the joints, in particular in patients suffering from RA or AS.

One outcome of the administration of the compounds of the present invention to patients suffering from RA and/or AS may thus be reducing the pain experienced by the subject suffering from RA or AS relative to the pain experienced by the subject immediately before the administration of the compounds or combinations of the present invention. Another outcome of said treatment may be preventing the re-occurence of pain which has previously been reduced as a result of pharmaco- or other therapy. Another outcome of treatment may be decreasing the occurrence of and/or the severity of manifestations related to an inflammatory autoimmune disease of the joints, including particularly RA and AS. The treatment may suitably result in an improved functionality of the joints, such as decreased stiffness, improved mobility.

The term "RA" refers to Rheumatoid Arthritis. RA is a chronic inflammatory autoimmune disease that causes the immune system to attack the joints, and particularly the synovium in the joint. The synovium becomes inflamed and causes swelling and pain. Cardinal symptoms of RA are joint pain and stiffness but additional symptoms include muscle aches, anemia and fever. Diagnosis of RA can be confirmed by detecting an antibody in the blood called the "rheumatic (or "rheumatoid") factor" and/or by a blood sedimentation test. Other useful and common tests are the detection of the "antinuclear antibody" or the "C-reactive protein".

"AS" stands for Ankylosing Spondylitis, which is a chronic, progressive autoimmune disease characterized by arthritis, inflammation and eventual immobility of the joints, particularly the spinal joints. It causes pain and stiffness in the back (often in the morning hours) as a result of ongoing swelling and irritation of the spinal joints (vertebrae). Inflammation of the tendons and ligaments that connect and provide support to the vertebrae can lead to pain and tenderness in the ribs, shoulder blades, hips, thighs, shins, heels and along the bony points of the spines.

Treatment of "non-inflammatory musculoskeletal pain" refers to the administration of the compounds or combinations of the compounds of the present invention to reduce the pain experienced by a subject suffering from non-inflammatory musculoskeletal pain including back pain, fibromyalgia, and myofascial pain syndrome. One outcome of treatment may be reducing the pain experienced by the subject relative to the pain experienced by the subject immediately before the administration of the compounds or combinations of the present invention. Another outcome of treatment may be preventing reoccurence of pain which has previously been reduced as a result of pharmacotherapy. Another outcome of treatment may be decreasing the occurrence of and/or the severity of manifestations related to non-inflammatory musculoskeletal pain including back pain, fibromyalgia, and myofascial pain syndrome. The treatment may suitably result in a reduction of increased muscle sensitivity characterized by pain evoked by a normally non-nociceptive stimulus (allodynia) or increased pain intensity evoked by nociceptive stimuli (hyperalgesia). Finally, the treatment of non-inflammatory musculoskeletal pain can also improve the associated symptoms of back pain, fibromyalgia, and myofascial pain syndrome.

The terms "fibromyalgia" or "FMS" relates to a syndrome that causes widespread pain and stiffness throughout the tissue that supports and moves bones and joints. Fibromyalgia can be diagnosed by the presence of excessive tenderness on applying pressure to at least 11 of 18 specific muscle-tendon sites.

"Myofascial pain syndrome" is a chronic non-degenerative, non-inflammatory musculoskeletal pain condition. Distinct areas within muscles or their delicate connective tissue coverings (fascia) become abnormally thickened or tight. When the myofascial tissues tighten and lose their elasticity, neurotransmitter ability to send and receive messages between the brain and body is damaged. Symptoms include muscle stiffness and aching and sharp shooting pains or tingling and numbness in areas distant from the trigger point. Most commonly trigger points are in the neck, back or buttocks.

"Back pain" is a common non-inflammatory musculoskeletal pain condition that may be either acute or chronic. It may be caused by a variety of diseases and disorders that affect the lumbar spine. Low back pain is often accompanied by sciatica, which is pain that involves the sciatic nerve and is felt in the lower back, the buttocks, and the backs of the thighs.

The compounds of the present invention are also useful for treating signs and symptoms of an overactive bladder such as urinary incontinence, more specific urinary urge incontinence, urinary stress incontinence, urinary urgency, nocturia and/or urinary frequency.

The pharmaceutical compositions according to the present invention are preferably adapted for oral administration. Alternatively, if skin diseases are to be treated the pharmaceutical composition containing the inventive compounds may be also formulated for topical or transcutaneous use.

In another aspect, the present invention relates to a method for inhibiting vanilloid ligand from binding to vanilloid receptor in a patient, comprising contacting cells expressing vanilloid receptor in the patient with a compound of formula (l), an isomer thereof, or a pharmaceutically acceptable salt thereof.

In another aspect, the present invention relates to a method for preventing or treating a disease selected from pain, migraine, arthralgia, neuropathies, nerve injury, skin disorder, urinary bladder hypersensitiveness, irritable bowel syndrome, fecal urgency, a respiratory disorder, irritation of skin, eye or mucous membrane, stomach-duodenal ulcer, inflammatory diseases, which comprises administering to a mammar including a person in need thereof a therapeutically effective amount of a compound of formula (l), an isomer thereof, or a pharmaceutically acceptable salt thereof.

In the above method, the disease is also selected from acute pain, chronic pain, neuropathic pain, post-operative pain, diabetic neuropathy, neurodegeneration, stroke, neurotic/allergic/inflammatory skin disease, psoriasis, pruritus, prurigo, asthma, chronic obstructive pulmonary disease, urinary incontinence or inflammatory bowel disease.

In one preferred aspect of the invention, the above method is treating pain that is or that is associated with a condition selected from osteoarthritis ("OA"), rheumatoid arthritis ("RA"), Ankylosing Spondylitis ("AS"), diabetic neuropathic pain, non-inflammatory musculoskeletal pain (including fibromyalgia, myofascial pain syndrome and back pain), post-operative pain, migraine and.other types of headache.

In another aspect, the present invention relates to the use of a compound of formula (l), an isomer thereof, or a pharmaceutically acceptable salt thereof as an antagonist of vanilloid receptor.

In another aspect, the present invention relates to the use of a compound of formula (l), an isomer thereof, or a pharmaceutically acceptable salt thereof for prevention or treatment of a condition related to vanilloid receptor, which is more specifically associated with the aberrant expression and/or aberrant activation of a vanilloid receptor.

In another aspect, the present invention relates to the use of a compound of formula (l), an isomer thereof, or a pharmaceutically acceptable salt thereof, in preparation of a medicament for prevention or treatment of a condition related to vanilloid receptor.

In a preferred aspect, the present invention relates to the use of a compound of formula (l), an isomer thereof, or a pharmaceutically acceptable salt thereof in the preparation of a medicament for the prevention or the treatment of a condition that is selected from pain, inflammatory autoimmune disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, COPD, pruritus or prurigo.

In a particularly preferred aspect, the present invention relates to the use of a compound for treating pain as described above, wherein the pain is or is associated with a condition that is selected from osteoarthritis ("OA"), rheumatoid arthritis ("RA"), Ankylosing Spondylitis ("AS"), diabetic neuropathic pain, post-operative pain, non-inflammatory musculoskeletal pain (including fibromyalgia, myofascial pain syndrome and back pain), migraine and other types of headaches.

Hereinafter, the formulating methods and kinds of excipients will be described, but the present invention is not limited to them.

A compound of formula (l), an isomer thereof or a pharmaceutically acceptable salt thereof according to the present invention can be prepared as a pharmaceutical composition containing pharmaceutically acceptable carriers, adjuvants, diluents and the like. For instance, the compounds of the present invention can be dissolved in oils, propylene glycol or other solvents which are commonly used to produce an injection. Suitable examples of the carriers include physiological saline, polyethylene glycol, ethanol, vegetable oils, isopropyl myristate, etc., but are not limited to them. For topical administration, the compounds of the present invention can be formulated in the form of ointment or cream.

The compound according to the present invention may also be used in the forms of pharmaceutically acceptable salts thereof, and may be used either alone or in combination or in admixture with other pharmaceutically active compounds.

The compounds of the present invention may be formulated into injections by dissolving, suspending or emulsifying in water-soluble solvent such as saline and 5% dextrose, or in water-insoluble solvents such as vegetable oils, synthetic fatty acid glyceride, higher fatty acid esters and propylene glycol. The formulations of the invention may include any of conventional additives such as dissolving agents, isotonic agents, suspending agents, emulsifiers, stabilizers and preservatives.

The preferable dose level of the compounds according to the present invention depends upon a variety of factors including the condition and body weight of the patient, severity of the particular disease, dosage form, and route and period of administration, but may appropriately be chosen by those skilled in the art. The compounds of the present invention are preferably administered in an amount ranging from 0.001 to 100 mg/kg of body weight per day, and more preferably from 0.01 to 30 mg/kg of body weight per day. Doses may be administered once a day, or several times a day with each divided portions. The compounds of the present invention are used in a pharmaceutical composition in an amount of 0.0001 ~ 10% by weight, and preferably 0.001 ~ 1% by weight, based on the total amount of the composition.

The pharmaceutical composition of the present invention can be administered to a mammalian subject such as rat, mouse, domestic animals, human being and the like via various routes. The methods of administration which may easily be expected include oral and rectal administration; intravenous, intramuscular, subcutaneous, intrauterine, duramatral and intracerebroventricular injections.

### Detailed description of the invention definitions

When describing the compounds, pharmaceutical compositions containing such compounds, methods of using such compounds and compositions, and use of such compounds and compositions, all terms used in the present application shall have the meaning usually employed by a relevant person skilled in the art, e.g. by a medicinal chemists, pharmacist or physician. By the way of example some definitions of specific groups are given below:

"Alkyl" includes monovalent saturated aliphatic hydrocarbyl groups. The hydrocarbon chain may be either straight-chained or branched. This term is exemplified by groups such as methyl, ethyl, n-propyl, isopropyl, n-butyl, iso-butyl, tert-butyl, and the like.

"Alkylcarbonyl" includes a carbonyl group linked to a alkyl group defined above and specifically includes, for example, methylcarbonyl (acetyl), ethylcarbonyl, n-propylcarbonyl, isopropylcarbonyl, and the like.

"Alkoxy" includes the group-OR where R is alkyl. Particular alkoxy groups include, by way of example, methoxy, ethoxy, n-propoxy, isopropoxy, n-butoxy, tert-butoxy, sec-butoxy, n-pentoxy, 1,2-dimethylbutoxy, and the like.

"Alkoxycarbonyl" includes the monovalent radical ROC(O)-, wherein R is alkyl as described above. Particular alkoxycarbonyl groups include, by way of example, methoxycarbonyl, ethoxycarbonyl, propoxycarbonyl, isopropoxycarbonyl, and the like.

"Alkenyl" includes monovalent olefinically unsaturated hydrocarbyl groups being straight-chained or branched and having at least 1 double bond. Particular alkenyl groups include ethenyl (-CH=CH₂), n-propenyl (-CH₂CH=CH₂), isopropenyl (-C (CH₃) =CH₂), and the like. A preferred "alkenyl" group is ethenyl (vinyl).

"Alkynyl" includes acetylenically unsaturated hydrocarbyl groups being straight-chained or branched and having at least 1 triple bond. A preferred alkynyl group is ethynyl (acetylene).

"Alkylsulfonyl" includes a radical-S(O)₂R where R is an alkyl group as defined herein. Representative examples include, but are not limited to, methylsulfonyl, ethylsulfonyl, propylsulfonyl, butylsulfonyl and the like.

"Alkylthio" includes a radical-S-R where R is an alkyl group as defined herein that may be optionally substituted as defined herein. Representative examples include, but are not limited to, methylthio, ethylthio, propylthio, butylthio, and the like.

"Amino" refers to the radical -NH₂.

"Carboxy" refers to the radical -C(=O)OH.

"Cyano" refers to the radical -CN.

"Cycloalkyl" refers to a nonaromatic monovalent hydrocarbon radical having preferably from three to eight carbon atoms. Typical cycloalkyl groups include, cyclopropyl, cyclobutyl, cyclopentyl, cyclohexyl, and the like.

"Ethenyl" refers to -CH=CHₛ which in the present application is also designated "vinyl"

"Ethynyl" refers to -C=CH.

"Halo" or "halogen" refers to fluoro, chloro, bromo and iodo. Preferred halo groups are either fluoro or chloro.

"Haloalkyl" includes an "alkyl" group as defined further above which is substituted with one or more halogens which may be the same, e.g. in trifluoromethyl, or which may be different

"Hydroxy" refers to the radical-OH.

"Nitro" refers to the radical-NO₂.

"Pharmaceutically acceptable" means approved by a regulatory agency of the Federal or a state government or listed in the U. S. Pharmacopoeia or other generally recognized pharmacopoeia for use in animals, and more particularly in humans.

"Pharmaceutically acceptable salt" refers to a salt of a compound of the invention that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3- (4-hydroxybenzoyl) benzoic acid, cinnamic acid, mandelic acid, methanesulfonic acid, ethanesulfonic acid, 1,2-ethanedisulfonic acid, 2hydroxyethanesulfonic acid, benzenesulfonic acid, 4-chlorobenzenesulfonic acid, 2naphthalenesulfonic acid, 4-toluenesulfonic acid,camphorsulfonic acid, 4methylbicyclo [2.2.2]-oct-2-ene-1-carboxylic acid, glucoheptonic acid,3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced.

"Pharmaceutically acceptable carrier" refers to a diluent, adjuvant, excipient or carrier with which a compound of the invention is administered.

"Preventing" or "prevention" refers to a reduction in risk of acquiring a disease or disorder (i. e., causing at least one of the clinical symptoms of the disease not to develop in a subject that may be exposed to or predisposed to the disease but does not yet experience or display symptoms of the disease).

"Subject" includes humans. The terms "human", "patient" and "subject" are used interchangeably herein.

"Therapeutically effective amount" means the amount of a compound that, when administered to a subject for treating a disease, is sufficient to effect such treatment for the disease. The "therapeutically effective amount" can vary depending on the compound, the disease and its severity, and the age, weight, etc., of the subject to be treated.

"Treating" or "treatment" of any disease or disorder refers, in one embodiment, to ameliorating the disease or disorder (i. e., arresting or reducing the development of the disease or at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (e. g., stabilization of a discernible symptom), physiologically, (e. g., stabilization of a physical parameter), or both. In yet another embodiment, "treating" or "treatment" refers to delaying the onset of the disease or disorder.

### Mode for carrying out invention

The present invention is more specifically explained by following examples and experimental examples. However, it should be understood that the extent of the present invention is not limited to the following examples and experimental examples.

### Example 1: N-{4-[3-(4-t-butylbenzyl)thioureido-methyl]-2-vinyl-phenyl} methanesulfonamide

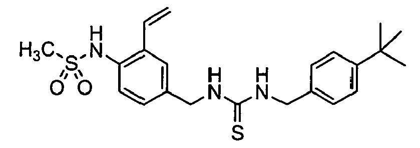

### Step 1: (4-nitrobenzyl)carbamic acid t-butyl ester

4-nitrobenzylamine HCl (1 g, 5.302 mmol, 1 *eq*.) was put into 100ml of round-bottom flask and dissolved in the saturated solution (NaHCO₃: CH₂Cl₂= 1: 1). To the solution was added di-t-butyl dicarbonate (3.66 ml, 15.906 mmol, 3 e*q*.) and stirred for 3 hours. After confirming the completion of the reaction with TLC, the reaction solution was extracted with methylenechloride, washed with water (twice) and brine, dried over Na₂SO₄, filtered and concentrated under reduced pressure. The obtained liquid was column-chromatographed (n-hexane /ethyl acetate = 10/1) to yield a pale light green solid (1.3171g).
IR (KBr pellet, cm⁻¹): 3330, 3080, 301, 2984, 2916, 1687, 1513;
¹H NMR (400 MHz, CDCl₃): 8.11 (d, 2H, J=8.4Hz), 7.37 (d, 2H, J = 8.4 Hz), 4.99 (bs, 1 H), 4.37 (d, 2H, J = 5.6 Hz), 1.39 (s, 9H).

### Step 2 : (4-aminobenzyl)carbamic acid t-butyl ester

(4-nitrobenzyl) carbamic acid t-butyl ester (1.3071g, 5.880mmol, 1eq.) was put into 100ml of round-bottom flask and dissolved in methanol. While adding Pd/c (about 10wt% of substrate) little by little, the reaction mixture was stirred for 2 hours at room temperature under hydrogen atmosphere. After confirming the progress of the reaction with TLC, the reaction solution was filtered through celite and evaporated to yield a pale yellow solid (1.1451g).
IR (KBr pellet, cm-¹): 3426, 3346, 3021, 2995, 2976, 1687.
¹H NMR (400 MHz, CDCl₃): 7.09 (d, 2H, J = 8.0 Hz), 6.70 (d, 2H, J = 8.0 Hz), 4.74 (bs, 1 H), 4.20 (d, 2H, J = 5.0 Hz), 3.34 (bs, 2H), 1.46 (s, 9H).

### Step 3 : (4-amino-3-iodobenzyl)carbamic acid t-butyl ester

A 100 ml of two-neck round bottom flask was filled with argon gas, and the solution of (4-aminobenzyl) carbamic acid t-butyl ester (986.2mg, 3.909mmol, 1*eq*.) in methylenechloride was put into the flask. To the solution was added iodomonochloride (698.2mg, 4.300mmol, 1.1 eq.) and stirred for 1 hour. After confirming the completion of the reaction with TLC, to the solution was added saturated sodium thiosulfate solution, and the mixture was stirred. The reaction solution was extracted with methylenechloride, washed with water (twice) and brine, dried over Na₂SO₄, filtered and concentrated under reduced pressure. The obtained liquid was column-chromatographed (n-hexane /ethyl acetate = 3/1) to yield a brown liquid (640mg).
IR (NaCl neat, cm⁻¹) : 3421, 3349, 2976, 2929, 1695 ;
¹H NMR (400 MHz, CDCl₃): 7.49 (d, 1H, J=2.0Hz), 6.98 (dd, 1H, J=8.0, 2.0Hz), 6.61 (d, 1H, *J*=8.0Hz), 4.87 (bs, 1 H), 4.07 (d, 2H, J=4.0Hz), 3.98 (bs, 2H), 1.40 (s, 9H)

### Step 4 : (4-amino-3-vinylbenzyl)carbamic acid t-butyl ester

A 50 ml of two-neck round bottom flask was filled with argon gas, and the solution of tetrakis(triphenyl phosphine)palladium(0) (123.7 mg, 0.107 mmol, 0.06 *eq.)* and lithium chloride(211.9mg, 4.998 mmol, 2.8 eq.) in DMF was put into the flask. To the solution were added (4-amino-3-iodobenzyl) carbamic acid t-butyl ester (621.4 mg, 1.875 mmol, 1 eq.) and tributylvinyltin (782.5mg, 2.678 mmol, 1.5 *eq*.). The mixture solution was heated to 90°C to reflux for one night. After confirming the completion of the reaction with TLC, the reaction solution was extracted with methylene chloride, washed with water and brine, dried over Na₂SO₄, filtered and evaporated. The obtained liquid was column-chromatographed (n-hexane /ethyl acetate = 3/1) to yield a brown liquid.

### Step 5 : (4-methanesulfonylamino-3-vinylbenzyl)carbamic acid t-butyl ester

A 50 ml of two-neck round bottom flask was filled with argon gas, and the solution of (4-aminobenzyl) carbamic acid t-butyl ester (343.0 mg, 1.381 mmol, 1 e*q*.) in methylenechloride was put into the flask. To the solution was added methanesulfonic anhydride (264.7 mg, 1.519 mmol, 1.1 *eq*.) at 0°C, followed by adding pyridine (332.0µl, 4.413mmol, 2eq.) and stirring for 1 hour. After confirming the completion of the reaction with TLC, to the solution was added saturated NaHCO₃ solution and stirred for 5 minutes. The reaction solution was extracted with methylenechloride and washed with 5% HCl, saturated NaHCO₃ solution, water and brine. The resulting solution was dried over Na₂SO₄, filtered and evaporated. The obtained solid was column-chromatographed (n-hexane /ethyl acetate = 2/1) to yield a pale yellow solid (161.6mg).
IR (KBr pellet, cm⁻¹) : 3414, 3359, 3269, 3254, 3083, 2982, 2927, 1685 ;
¹H NMR (400 MHz, CDCl₃): 7.35( d, 1 H, *J*=8.0Hz), 7.33( d, 1 H, *J*=2.0Hz), 7.14( dd, 1 H, J=8.0, 2.0Hz), 6.84( dd, 1 H, J=17.2, 10.8Hz), 6.44( bs, 1 H), 5.65( d, 1 H, J=17.2Hz), 5.40( d, 1 H, J=10.8Hz), 4.84( bs, 1 H), 4.23( d, 2H, J=5.6Hz) , 2,91 ( s, 3H), 1.39( s, 9H)

### Step 6 : N-(4-aminomethyl-2-vinylphenyl)methanesulfonamide

(4-methanesulfonylamino-3-vinylbenzyl) carbamic acid t-butyl ester(161.6mg) was put into 100ml of round-bottom flask and dissolved in methylenechloride. To the solution was added trifluoroacetic acid and stirred for one night. After confirming the completion of the reaction with TLC, the reaction solution was concentrated under reduced pressure to yield a brown liquid (198.6mg).

### Step 7: N-{4-[3-(4-t-butylbenzyl)thioureidomethyl]-2-vinyl-phenyl}methanesulfonamide

N-(4-Aminomethyl-2-vinylphenyl)methanesulfonamide (50mg, 0.221mmol) and TEA (61.6µl, 0.442 mmol, 2eq.) were added in methylene chloride. After the reaction mixture was cooled down to 0°C, 1-*t*-*butyl*-4-isothiocyanatomethylbenzene (49.9mg, 0.243mmol, 1.1*equiv.)* was added into mixture. The reaction mixture was stirred overnight at room temperature. After confirming the completion of the synthesis, a reaction solvent was removed in vacuo. The residue was purified by column chromatography (n-Hx: EA= 1: 1) to yield a solid (40.3mg, 42.2%).
mp: 96-97 °C;
IR (KBr pellet, cm⁻¹): 3433, 3092, 2961, 2869, 1550, 1151;
¹H NMR(400MHz, CDCl₃) : 7.32( d, 1H, J=2.0Hz), 7.30-7.26( m, 3H), 7.13( d, 2H, J=8.4Hz), 7.07( dd, 1H, J=8.4, 2.0Hz), 6.80( dd, 1H, J=17.2, 11.2Hz), 6.48( s, 1H), 6.13( bs, 1 H), 5.62( dd, 1H, *J*=17.2, 0.8Hz), 5.39( dd, 1H, *J*=11.2, 0.8Hz), 4.59( s, 2H), 4.50( s, 2H), 2.88( s, 3H), 1.23( s, 9H)

### Example 2: N-{4-[3-(4-t-butylbenzyl)thioureido-methyl]-2-trifluoromethyl-6-vinyl-phenyl}methanesulfonamide

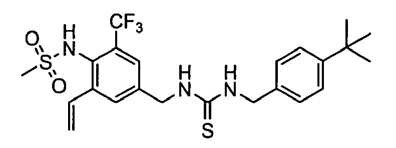

### Step 1: 4-lodo-2-trifluoromethylphenylamine

2-Trifluoromethylphenylamine (30mg, 0.21mmol) and ICI (1.1eq, 0.24mmol, 38.97 mg) were added into methylene chloride. The reaction mixture was stirred for 12hrs. The reaction mixture was purified according to step 2 of Example 8 to give the title compound (25.6mg, 42.48%).
IR (NaCl neat, cm⁻¹): 3411, 3066, 1109, 701;
¹H NMR (400MHz, CDCl₃): δ 7.61 (d, 1H, J=2.0Hz), 7.46(dd, 1H, J=8.4, 1.2Hz), 6.45(d, 1 H, J=8.4Hz), 4.05(bs, 2H).

### Step 2: 4-Amino-3-trifluoromethylbenzonitrile

4-lodo-2-trifluoromethylphenylamine (50mg, 0.17mmol), Zn(CN)₂ (0.88eq, 0.15mmol, 18.00mg), and Pd(PPh₃)₄(0.1eq, 0.02mmol, 19.64mg) were added DMF. Zn(CN)₂ (0.88eq, 0.15mmol, 18.00mg) and Pd(PPh₃)₄(0.1eq, 0.02mmol, 19.64mg) were added into the reaction mixture. The reaction mixture was stirred for 12hrs. The reaction mixture was purified according to step 1 of Example 8 to give the title product, as a yellow solid (31.4 mg, 99.28%).
mp: 54-56 °C;
IR (KBr pellet, cm⁻¹): 3385, 3263, 2924, 2220, 1124, 701
¹H NMR (400MHz, CDCl₃): δ 7.65(d, 1 H, J=2.0Hz), 7.45(dd, 1 H, J =8.4, 2.0Hz), 6.69(d, 1 H, J =8.4Hz), 4.65(s, 2H).

### Step 3: 4-Amino-3-iodo-5-trifluoromethylbenzonitrile

4-Amino-3-trifluoromethylbenzonitrile (100mg, 0.54mmol) and lCl(1.1eq, 0.58mmol, 96.00mg) were added to methylene chloride. The mixture was stirred for 12hrs. The reaction mixture was purified accoding to step 2 of Example 8 to give the title compound (30.5mg, 18.10%).
mp: 86~88 °C; IR(KBr pellet, cm⁻¹): 3371, 3080, 2924, 2226, 1125, 701; ¹H NMR(400MHz, CDCl₃): δ 8.02 (d, 1 H, J=1.6Hz), 7.35 (dd, 1 H, J=133.2, 1.6Hz), 7.65 (dd, 1H, J=10.0, 1.6Hz), 5.20 (d, 2H, J=22.0Hz).

### Step 4: (4-Amino-3-iodo-5-trifluoromethylbenzyl)carbamic acid t-butyl ester

4-Amino-3-iodo-5-trifluoromethylbenzonitrile (1805.2mg, 5.19mmol) was dissolved in THF at 0°C. After Borane-THF complex (3eq, 17.36mmol, 17.36ml) was slowly added into the reaction mixture, a reaction temperature was heated to reflux. The reaction mixture was stirred for 12hrs with reflux. After confirming the completion of the reaction, MeOH was added. The mixture was stirred for 4hrs. The reaction solvent was removed in vacuo. A residue was extracted with Ethyl acetate, washed with H₂O and brine, dried with Na₂SO₄ and concentrated in vacuo to yield 4-Aminomethyl-2-iodo-6-trifluoromethylphenylamine (55.1 mg).

4-Aminomethyl-2-iodo-6-trifluoromethyl-phenylamine (2025.7mg, 6.45mmol) was dissolved in THF, and then BOC₂O(0.8eq, 5.16mmol, 1187.15*µ*ℓ) was slowly added. The reaction mixture was stirred for 12hrs. A reaction mixture was extracted with Ethyl acetate, washed with H₂O and brine, dried over Na₂SO₄, concentrated in vavuo. A residue was purified by column chromatography (n-Hx: EA= 5: 1) to yield a solid (1501.1 mg, 55.94%).
mp: 120~122°C; IR(KBr pellet, cm⁻¹): 3387, 2984, 1687, 1107, 701;
¹H NMR(400MHz, CDCl₃): δ 7.43(d, 1H, J=160.4Hz), 7.27(d, 1 H, J=37.2Hz), 4.92(s, 1 H), 4.54(s, 2H), 4.13(s, 2H), 1.41 (s, 9H).

### Step 5: (4-Amino-3-trifluoromethyl-5-vinylbenzyl)carbamic acid t-butyl ester

Pd(PPh₃)₄ (0.06eq, 0.014mmol, 16.64mg) and LiCl (2.8eq, 0.67mmol, 28.49mg) were added into DMF. (4-Amino-3-iodo-5-trifluoromethylbenzyl)carbamic acid *t-butyl* ester (100mg, 0.24mmol) and tributylvinyltin (1.5eq, 0.36mmol, 105.37 µl) were added into the reaction mixure. The reaction mixture was stirred for 12 hrs in reflux. The reaction mixture was purified according to step 4 of Example 8 to give the title product, as a yellow syrup (51.5mg, 67.87%).
mp:90-92 °C; IR(NaCl neat, cm⁻¹): 3357, 2981, 1702, 1635, 1116;
¹H NMR(400MHz, CDCl₃): δ 7.24(s, 1 H), 7.22(s, 1H), 6.64(dd, 1 H, J=17.2, 6.4Hz), 5.57(dd, 1 H, J=17.2, 1.2Hz), 5.36(dd, 1 H, J=10.8, 1.2Hz), 4.71 (s, 1H), 4.15(s, 2H), 4.14(d, 2H J=5.2Hz), 1.39(s, 9H)

### Step 6: (4-Methanesulfonylamino-3-trifluoromethyl-5-vinylbenzyl)carbamic acid t-butyl ester

(4-Amino-3-trifluoromethyl-5-vinylbenzyl)carbamic acid *t-butyl* ester (235.6mg, 0.75mmol), methanesulfonyl chloride (5eq, 3.73mmol, 288.40*µ*ℓ), and triethylamine (3eq, 2.25mmol, 313.60*µ*ℓ) were added to methylene chloride. The reaction mixture was stirred for 5hrs. The reaction mixture was purified according to step 5 of Example 8 to give the title product, as a yellow syrup (89.3mg, 30.21%).
IR (KBr pellet, cm⁻¹): 3361, 2977, 1692, 1330, 1152;
¹H NMR(400MHz, CDCl₃): 7.65(s, 1H), 7.46(s, 1H), 7.13(dd, 1H, J=11.2, 17.6Hz), 6.09(s, 1 H), 5.73(d, 1 H, J=17.6Hz), 5.42(d, 1 H, J=11.2Hz), 4.90(s, 1 H), 4.29(d, 2H, J=5.6Hz), 3.07(s, 3H), 1.40(s, 9H)

### Step 7: N-(4-Aminomethyl-2-trifluoromethyl-6-vinylphenyl)methanesulfonamide

(4-Methanesulfonylamino-3-trifluoromethyl-5-vinylbenzyl)carbamic acid *t-butyl* ester (89.3mg, 0.23mmol) and CF₃COOH( 5-6 drops) were added into methylene chloride. The mixture was stirred for 12hrs. The reaction mixture was purified according to step 6 of Example 8 to give the title product (101.4mg, 100%).
¹H NMR(400MHz, CD₃OD): 8.02(s, 1H), 7.77(s, 1H), 7.27(dd, 1H, *J*=17.6, 11.2Hz), 5.92(d, 1 H, *J*=17.6Hz), 5.51 (d, 1 H, *J*=11.2Hz), 4.20(s, 2H), 3.11 (s, 3H).

### Step 8: N-{4-[3-(4-t-butylbenzyl)thioureidomethyl]-2-trifluoromethyl-6-vinylphenyl}methanesulfonamide

N-(4-Aminomethyl-2-trifluoromethyl-6-vinylphenyl)methanesulfonamide (57.8 mg, 0.18 mmol), 1-*t*-*butyl*-4-isothiocyanatomethylbenzene (1.1 eq, 0.19mmol, 45.28*µ*ℓ), and TEA(3eq, 0.54mmol, 75.27mg) were added in methylene chloride under argon atmosphere. The reaction mixture was stirred for 12hrs. The reaction solvent was concentrated in vacuo. The residue was extracted with methylene chloride. A combined organic layer was washed with H₂O and brine, dried over Na₂SO₄, and concentrated in vacuo. The residue was purified with column chromatography (n-Hx: EA= 2: 1) to yield the title product (50.04 mg, 55.69%).
mp:100~102°C;
IR( KBr pellet, cm⁻¹): 3358, 3268, 3058, 2963, 1550, 1328, 1150;
¹H NMR(400MHz, CDCl₃): δ 7.67(s, 1H), 7.44(s, 1 H), 7.30(d, 2H, J =8.4Hz), 7.15(d, 2H, J =8.4Hz), 7.09(dd, 1 H, J =17.6, 11.2Hz), 6.37(s, 1 H), 5.71 (d, 1 H, J =17.2Hz), 5.41 (d, 1 H, J =11.2Hz), 4.70(s, 2H), 4.52(bs, 2H), 3.05(s, 3H), 1.24(s, 9H)

### Example 3: N-{4-[3-(4-t-butylbenzyl)thioureido-methyl]-2-fluoro-6-vinyl-phenyl}methanesulfonamide

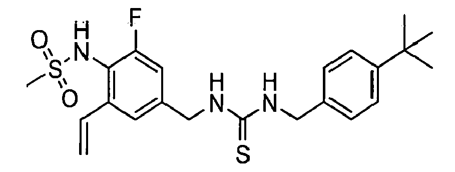

### Step 1 : 4-aminomethyl-2-fluoro-6-iodophenylamine

A 50 ml of two-neck round bottom flask was filled with argon gas, and the solution of 4-amino-3-fluoro-5-iodo-benzonitrile (84.4mg, 0.322mmol, 1*eq*.) in tetrahydrofuran was put into the flask, and then cooled to 0°C. To the solution was added Borane-THF complex solution (1.0M solution in THF, 0.64ml, 0.644mmol, 2eq.). The temperature of reaction mixture was raised to room temperature, and heated to reflux. After confirming the completion of the reaction with TLC, to the solution was added 5% HCl and stirred for 20 minutes. The resulting solution was basified using 1 N KOH, extracted with ether, washed with brine, and dried over Na₂SO₄. The obtained liquid was concentrated under reduced pressure to yield a pale yellow solid (78.4mg, 80.5%).
IR(KBr pellet, cm-¹) : 3429, 2923, 2853, 1626 ;
¹H NMR (400MHz, CD₃OD): 7.33(s, 1H), 6.93(dd, 1H, J=11.6, 2.0Hz), 3.58(s, 2H).

### Step 2 : (4-amino-3-fluoro-5-iodobenzyl)carbamic acid t-butyl ester

A 25 ml of two-neck round bottom flask was filled with argon gas, and the solution of 4-aminomethyl-2-fluoro-6-iodophenylamine (31.9mg, 0.120mmol, 1 eq.) and triethylamine (18.4*µ*ℓ, 0.132mmol, 1.1*eq*.) in methylenechloride were put into the flask, and then cooled to 0°C. To the solution were added 4-dimethylaminopyridine (1.47mg, 0.012mmol, 0.1*eq*.) and di-t-butyl dicarbonate (27.6*µ*ℓ, 0.120mmol, 1*eq*.), and stirred for 5 hours. After confirming the completion of the reaction with TLC, the resulting solution was extracted with methylenechloride, washed with water and brine, dried over Na₂SO₄, and concentrated under reduced pressure. The obtained liquid was column-chromatographed (n-hexane /ethyl acetate = 5/1) to yield a yellow liquid(9.8mg, 22.3%). IR (NaCl neat, cm⁻¹) : 3451, 3351, 2975, 2928, 1698 ;
¹H NMR (400 MHz, CDCl₃): 7.87 (s, 0.2H), 7.44 (d, 0.2H, *J*=11.2Hz), 7.26 (s, 1 H), 6.87 (d, 1 H, *J*=11.2Hz), 4.72 (bs, 2H), 4.08 (d, 2H, *J*=4.4Hz), 1.39 (s, 9H)

### Step 3 : (4-amino-3-fluoro-5-vinylbenzyl)carbamic acid t-butyl ester

A 25 ml of two-neck round bottom flask was filled with argon gas, and the solution of tetrakis (triphenyl phosphine) palladium(0) (18.9mg, 0.016mmol, 0.06eq.) and lithium chloride(32.4 mg, 0.765 mmol, 2.8 eq.) in DMF was put into the flask. To the solution were added (4-amino-3-fluoro-5-iodo-benzyl)-carbamic acid t-butyl ester (100 mg, 0.273 mmol, 1 eq.) and tributylvinyltin (119.7 *µ*ℓ, 0.410 mmol, 1.5 *eq*.), and heated to reflux for 5 hours. After confirming the completion of the reaction with TLC, the resulting solution was extracted with ethylacetate, washed with water and brine, dried over Na₂SO₄, and concentrated under reduced pressure. The obtained liquid was column-chromatographed (n-hexane /ethyl acetate = 5/1) to yield a brown liquid (52.5mg, 72.2%).
IR (NaCl neat, cm⁻¹) : 3412, 3088, 2958, 2925, 1689 ;
¹H NMR (400 MHz, CDCl₃): 7.53( d, 1H, *J*=2.0Hz), 7.39( dd, 1H, *J*=10.8, 2.0Hz), 6.64( dd, 1 H, J=17.6, 11.2Hz), 5.69( d, 1 H, *J*=17.6Hz), 5.42( d, 1 H, *J*=11.2Hz), 4.36( s, 2H), 1.49( s, 9H)

### Step 4 : (3-fluoro-4-methanesulfonylamino-5-vinylbenzyl)carbamic acid t-butyl ester

A 25 ml of two-neck round bottom flask was filled with argon gas, and the solution of (4-amino-3-fluoro-5-vinyl-benzyl)-carbamic acid t-butyl ester (27.3mg, 0.103mmol, 1*eq*.) in pyridine was put into the flask, and then cooled to 0°C. To the solution was added methanesulfonyl chloride (11.9*µ*ℓ, 0.154mmol, 1.5eq.), and heated to reflux for one night. After confirming the completion of the reaction with TLC, to the solution were added the solution (THF: H₂O= 2: 1) and NaOH (20.6mg, 0.515mmol, 5eq.), and stirred for 1 hour at room temperature. The reaction solution was acidified with 10% HCl, extracted with ethylacetate, washed with water and brine, dried over Na₂SO₄ a evaporated. The obtained solid was column-chromatographed (n-hexane /ethyl acetate = 2/1) to yield a yellow liquid.
IR (NaCl neat, cm⁻¹): 3349, 3236, 2956, 2921, 2850, 1689 ;
¹H NMR (400 MHz, CDCl₃): 7.27 (s, 1 H), 7.10 (dd, 1 H, J=17.6, 10.8Hz), 6.97( d, 1H, J=10.0Hz) , 5.88 (bs, 1 H), 5.73 (d, 1 H, J=17.6Hz), 5.39 (d, 1 H, J=10.8Hz), 4.87 (bs, 1 H), 4.25( d, 2H, J=6.0Hz) , 3.00( s, 3H), 1.40( s, 9H)

### Step 5 : N-(4-aminomethyl-2-fluoro-6-vinylphenyl)methanesulfonamide

(3-fluoro-4-methanesulfonylamino-5-vinyl-benzyl)-carbamic acid t-butyl ester (1ml) was put into 25ml of round-bottom flask, and dissolved in methylenechloride. To the solution was added trifluoroacetic acid (1ml) and stirred for one night. After confirming the completion of the reaction with TLC, the reaction solution was concentrated under reduced pressure to yield a brown crude liquid (236.7mg).

### Step6:N-{4-[3-(4-t-butylbenzyl)thioureidomethyl]-2-fluoro-6-vinylphenyl}methanesulfonamide

N-(4-Aminomethyl-2-fluoro-6-vinylphenyl)methanesulfonamide (40.8 mg, 0.187 mol), 1*-t-butyl-*4-isothiocyanatomethylbenzene (48.3 mg, 0.205 mol), and TEA (78.2 µl, 0.561 mmol, 3eq) were added in methylene chloride under argon atmosphere. The reaction mixture was stirred overnight at room temperature. The mixture was poured in CH₂Cl₂. The reaction mixture was washed with H₂O and brine, dried over Na₂SO₄, and concentrated in vacuo. The residue was purified with column chromatography(n-Hexane: EtOAc= 1: 1) to give the title product (67.7 mg, 80.6%) as a solid.
Melting point (°C): 96-98;
IR (KBr pellet, cm⁻¹): 3418, 3057, 2962, 1550, 1312, 1152 ;
¹H NMR(400MHz, CDCl₃) : 7.25(d, J=8.4Hz, 2H), 7.19(s, 1H), 7.11(d, J=8.0Hz, 1H), 6.97(dd, J=17.6, 10.8Hz, 1H), 6.85(d, J=9.6Hz, 1H), 6.52(bs, 2H), 5.64(d, J=17.6Hz, 1 H), 5.31 (d, J=10.8Hz, 1 H), 4.57(s, 2H), 4.46(s, 2H), 2.88(s, 3H), 1.20(s, 9H)

### Example 4: N-{4-[3-(4-t-butylbenzyl)thioureido-methyl]-2-chloro-6-vinyl-phenyl}methanesulfonamide

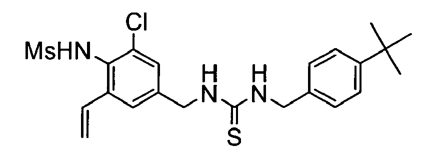

### Step 1: 4-Amino-3-chloro-5-iodobenzonitrile

4-Amino-3-chloro-benzonitrile (100mg, 0.66mmol) and ICI (1.1eq, 0.72mmol, 117.05mg) was added. The reaction mixture was stirred for 12hrs. The reaction mixture was purified according to the similar procedure to step 2 of Example 40 to obtain 4-Amino-3-chloro-5-iodobenzonitrile (65.2mg, 35.80%).
Mp: 121~123 °C;
IR( KBr pellet, cm⁻¹): 3365, 2942, 2221,1634, 728;
¹H NMR(400MHz, CDCl₃): δ 7.42(d, 1 H, J=1.6Hz), 7.43(d, 1 H, J=1.6Hz), 5.01 (bs, 2H).

### Step 2: (4-Amino-3-chloro-5-iodobenzyl)carbamic acid t-butyl ester

4-Amino-3-chloro-5-iodobenzonitrile (65.2mg, 0.23mmol) was dissolved in THF at 0°C. After Borane-THF complex (4eq, 0.94mmol, 0.94ml) was slowly added into the reaction mixture. A reaction temperature was heated to reflux. The reaction mixture was stirred for 12hrs with reflux. After confirming the completion of the reaction, MeOH was added. The mixture was stirred for 4hrs. The reaction solvent was removed in vacuo. A residue was extracted with Ethyl acetate, washed with H₂O and brine, dried over Na₂SO₄ and concentrated in vacuo to yield 4-Aminomethyl-2-iodo-6-methylphenylamine (19.4mg).
4-Aminomethyl-2-chloro-6-iodophenylamine (52.92mg, 0.19mmol) was dissolved in THF, and then BOC₂O (1.1eq, 0.21mmol, 47.48ml) was slowly added. The reaction mixture was stirred for 12hrs. A reaction mixture was extracted with Ethyl acetate, washed with H₂O and brine, dried over Na₂SO₄, and concentrated in vavuo. A residue was purified with column chromatography (n-Hx: EA= 5: 1) to yield a solid (34.37mg, 47.94%).
Mp: 113~115°C;
IR (KBr pellet, cm⁻¹): 3343, 1615, 717;
¹H NMR (400MHz, CDCl₃): δ 7.39(s, 1H), 7.09(s, 1H), 4.76(bs, 1H), 4.05(bs, 2H), 4.05(s, 2H), 1.38(s, 9H)

### Step 3: (4-Amino-3-chloro-5-vinylbenzyl)carbamic acid t-butyl ester

Pd(PPh₃)₄ (0.06eq, 0.01 mmol, 18.15mg) and LiCl (2.8eq, 0.73mmol, 30.86mg) was dissolved in DMF. 4-Amino-3-chloro-5-iodobenzyl)carbamic acid *t-butyl* ester (100mg, 0.26mmol) and tributylvinyltin (1.5eq, 0.39mmol, 114.76*µ*ℓ) were added. The reaction mixture was stirred for 12hrs in reflux. A reaction mixture was purified according to a similar procedure to step 3 to obtain a solid (61.8mg, 85.34%).
Mp: 85-87°C;
IR (KBr pellet, cm⁻¹): 3316, 2977, 1702, 1635, 725;
¹H NMR(400MHz, CDCl₃): δ7.06(d, 1H, *J*=2.0Hz), 7.01(d, 1H, *J*=2.0Hz), 6.64(dd, 1H, *J*=17.6, 11.2Hz), 5.56(dd, 1H, *J*=17.2, 1.2Hz), 5.30(dd, 1H, *J*=11.2, 1.2Hz), 4.72(bs, 1 H), 4.11 (d, 2H, J=5.2Hz), 1.38(s, 9H).

### Step 4: (3-Chloro-4-methanesulfonylamino-5-vinylbenzyl)carbamic acid t-butyl ester

(4-Amino-3-chloro-5-vinylbenzyl)carbamic acid *t-butyl* ester (40.2mg, 0.14mmol), methanesulfonyl chloride(5eq, 0.71 mmol, 55.15*µ*ℓ), and triethylamine(3eq, 0.42mmol, 58.34*µ*ℓ) were added in methylene chloride. The reaction mixture was stirred for 5hrs. The reaction mixture was purified with the similar procedure to step 5 of Example 8 to obtain the title compound (30.7mg, 59.83%).
Mp: 133-135°C;
IR( KBr pellet, cm⁻¹): 3353, 2981, 1691, 1524, 1322, 740;
¹H NMR(400MHz, CDCl₃): δ 7.40(s, 1 H), 7.25(d, 1 H, J=1.2Hz), 7.19(d, 1 H, J=1.2Hz), 7.14(dd, 1H, J=17.6, 11.2Hz), 6.18(s, 1H), 5.70(d, 1H, J=17.2Hz), 5.34(d, 1H, J=11.2Hz), 4.88(s, 1 H), 4.24(d, 2H, J=6.0Hz), 3.00(s, 3H), 1.40(s, 9H).

### Step 5: N-(4-Aminomethyl-2-chloro-6-vinylphenyl)methanesulfonamide

(3-Chloro-4-methanesulfonylamino-5-vinylbenzyl)carbamic acid *t-butyl* ester (30.7mg, 0.09mmol) and CF₃COOH (5-6 drops) was added into methylene chloride. The reaction mixture was stirred for 12hrs. The reaction mixture was concentrated in vacuo to give the title compound (33.4mg, 100%).
¹H NMR (400MHz, CD₃OD): δ7.75(d, 1 H, J=1.6Hz), 7.55(d, 1H, J=2.0Hz), 7.22(dd, 1H, J=17.6, 10.8Hz), 5.88(d, 1H, J=17.6Hz), 5.43(d, 1H, J=11.2Hz), 4.13(s, 2H), 3.10(s, 3H)

### Step6:N-{4-[3-(4-t-butylbenzyl)thioureidomethyl]-2-chloro-6-vinylphenyl}methanesulfonamide

N-(4-Aminomethyl-2-chloro-6-vinylphenyl)methanesulfonamide (25.4 mg, 0.10 mmol), 1-*t*-butyl-4-isothiocyanatomethylbenzene (1.1eq, 0.11 mmol, 25.85 mg), and TEA (3eq, 0.30 mmol, 41.81 *µ*ℓ) were added in methylene chloride under argon atmosphere. The reaction mixture was stirred for 12hrs. The reaction mixture was purified with column chromatography(n-Hx: EA= 2: 1) to yield the title product (20.8 mg, 44.71%) as a white solid.
Melting point: 106~108 °C;
IR( KBr pellet, cm⁻¹): 3358, 3261, 3026, 2963, 1652, 1323;
¹H NMR(400MHz, CDCl₃): δ 7.43(s, 1H), 7.33(d, 2H, *J* =8.8Hz), 7.19(d, 2H, *J* =8.8Hz), 7.13(dd, 1H, *J* =17.6, 11.2Hz), 6.28(s, 1H), 5.71(d, 1H, *J* =17.6Hz), 5.37(d, 1H, *J* =11.2Hz), 4.69(s, 2H), 4.56(s, 2H), 3.02(s, 3H), 1.26(s, 9H)

### Example 5: N-{4-[3-(4-t-butylbenzyl)thioureido-methyl]-2-methyl-6-vinyl-phenyl}methanesulfonamide

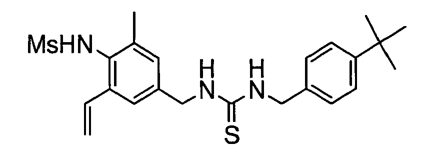

### Step 1: 4-Amino-3-methylbenzonitrile

4-iodo-2-methylaniline (2000mg, 8.58mmol) and cyanide(1.15g, 12.87mmol, 1.5eq)were added to pyridine. A reaction mixture was heated to 150~160 °C and stirred for 12hrs. A reaction mixture was diluted with methylene chloride. A diluted solution was washed with copper sulfate for several times. A mixture was washed with H₂O (2 times) and brine, and then dried over Na₂SO₄. A residue was purified with column chromatography (n-Hexane : EtOAc =2 : 1) to yield a solid (786.5mg, 69.34%).
Mp: 78-80 °C;
IR(NaCl neat, cm-¹): 3403, 3335, 3220, 2942, 2220;
¹H NMR(400MHz, CDCl₃): 7.24(m, 2H), 6.57(d, 1 H, J=8.4Hz), 4.03(bs, 2H), 2.08(s, 9H).

### Step 2: 4-Amino-3-iodo-5-methylbenzonitrile

4-Amino-3-methyl-benzonitrile (786.5 *µ*ℓ, 5.95mmol) and ICI(1.1eq, 6.55mmol, 1.06g) were added to methylene chloride. A reaction mixture was stirred for 12hrs. A reaction mixture was quenched by adding sodium thiosulfate solution. A aqueous solution was extracted with MC. A combined organic solution was washed with H₂O and brine, dried over Na₂SO₄ and concentrated in vacuo. A residue was purified with column chromatography(n-Hx: EA= 3:1) to yield a solid (600.6mg, 39.11%).
Mp: 131-133°C;
IR( KBr pellet, cm⁻¹): 3462, 3366, 2923, 2214, 1623;
¹H NMR(400MHz, CDCl₃): δ7.22(d, 1H, J=2.0Hz), 7.21(m, 1H), 2.16(s, 3H)

### Step 3: (4-Amino-3-iodo-5-methylbenzyl)carbamic acid t-butyl ester

4-Amino-3-iodo-5-methylbenzonitrile (200mg, 0.77mmol) was dissolved in THF at 0°C. After borane-THF complex (4eq, 3.10mmol, 3.10ml) was slowly added into the reaction mixture. A reaction temperature was heated to reflux. The reaction mixture was stirred for 12hrs with reflux. After confirming the completion of the reaction, MeOH was added. The mixture was stirred for 4hrs. The reaction solvent was removed in vacuo. A residue was extracted with Ethyl acetate, washed with H₂O and brine, dried over Na₂SO₄ and concentrated in vacuo to yield 4-Aminomethyl-2-iodo-6-methylphenylamine (194mg).

4-Aminomethyl-2-iodo-6-methyl-phenylamine (195mg, 0.76mmol) was dissolved in THF, and then BOC₂O (1.1eq, 0.21mmol, 47.48ml) was slowly added. The reaction mixture was stirred for 12hr. A reaction mixture was extracted with Ethyl acetate, washed with H₂O and brine, dried with Na₂SO₄, concentrated in vacuo. A residue was purified with column chromatography (n-Hx: EA= 5: 1) to give a solid (73.2mg, 34.77%).
Mp: 135-137°C;
IR (KBr pellet, cm⁻¹): 3354, 2995, 1675, 1617, 726;
¹H NMR (400MHz, CDCl₃): δ7.38 (s, 1 H), 6.90 (s, 1 H), 4.78 (s, 1H), 4.08 (d, 2H, J=5.2Hz), 4.01 (bs, 2H), 2.16 (s, 3H), 1.42 (s, 9H)

### Step 4: (4-Amino-3-methyl-5-vinylbenzyl)carbamic acid t-butyl ester

Pd(PPh₃)₄(0.06 eq, 0.017 mmol, 19.41mg) and LiCl (2.8 eq, 0.74 mmol, 33.23 mg) were dissolved in DMF. (4-Amino-3-iodo-5-methylbenzyl)carbamic acid t-butyl ester (100mg, 0.28mmol) and Tributylvinyltin (1.5eq, 0.41 mmol, 121.08*µ*ℓ) were added. The reaction mixture was stirred for 12hrs in reflux. A reaction mixture was purified according to a similar procedure to step 3 to obtain a solid (61.8mg, 85.34%).
IR (NaCl neat, cm⁻¹): 3373, 2965, 1697, 1632;
¹H NMR(400MHz, CDCl₃): δ 6.99(s, 1 H), 6.85(s, 1 H), 6.69(dd, 1 H, J=17.2, 10.8Hz), 5.53(dd, 1 H, J=17.2, 1.6Hz), 5.24(dd, 1 H, J=10.8, 1.6Hz), 4.68(bs, 1H), 4.10(d, 2H, J=5.2Hz), 3.70(bs, 2H), 2.08(s, 3H), 1.38(s, 9H)

### Step 5: (4-Methanesulfonylamino-3-methyl-5-vinylbenzyl)carbamic acid t-butyl ester

(4-Amino-3-methyl-5-vinyl-benzyl)-carbamic acid t-butyl ester (30.9mg, 0.12mmol), methanesulfonyl chloride(10 eq, 1.2 mmol, 91 *µ*ℓ) and Triethylamine(6 eq, 0.36mmol, 50.17) were added to methylene chloride. The reaction mixture was stirred for 12hrs. A reaction mixture was purified with a similar procedure to step 4 of example 8 to obtain a syrup (9.5mg, 23.70%).
IR (NaCl neat, cm⁻¹): 3371, 2961, 1697, 1513, 1316;
¹H NMR(400MHz, CDCl₃): δ 7.27(s, 1H), 7.06(s, 1H), 7.01 (dd, 1H, J=17.6, 11.2Hz), 5.80(s, 1 H), 5.68(dd, 1 H, J=17.7, 0.8Hz), 5.33(dd, 1 H, J=11.2, 0.8Hz), 4.79(s, 1 H), 4.22(d, 2H, J=6.0Hz), 2.98(s, 3H), 2.36(s, 3H), 1.40(s, 9H).

### Step 6: N-(4-Aminomethyl-2-methyl-6-vinyl-phenyl)-methanesulfonamide

(4-Methanesulfonylamino-3-methyl-5-vinylbenzyl)carbamic acid *t-butyl* ester (85.9mg, 0.09mmol) was dissolved in CH₂Cl₂. 5~6 drops of CF₃COOH were added. The reaction mixture was stirred for 12hrs. The reaction mixture was concentrated to yield a brownish syrup (100.4mg).
¹H NMR(400MHz, CD₃OD): δ 7.57(d, 1H, J=1.6Hz), 7.28(d, 1H, J=1.6Hz), 7.17(dd, 1H, J=17.6, 10.8Hz), 5.03(d, 1 H, J=17.6Hz), 5.38(d, 1H, J=10.8Hz), 4.06(s, 2H), 3.00(s, 3H), 2.41 (s, 3H)

### Step 7: N-{4-[3-(4-t-butylbenzyl)thioureidomethyl]-2-methyl-6-vinylphenyl}methanesulfonamide

N-(4-Aminomethyl-2-methyl-6-vinylphenyl)methanesulfonamide (32.9 mg, 0.14 mmol), 1-*t-butyl-*4-isothiocyanatomethylbenzene (1.1 eq, 0.15 mmol, 35.44 mg) and TEA (3eq, 0.42mmol, 58.54 µl) were added in methylene chloride under argon atmosphere. The reaction mixture was stirred for 12 hrs. The reaction mixture was purified with column chromatography (n-Hx: EA= 2: 1) to yield the title product (29.5 mg, 47.33%) as a white solid.
Melting point: 94~96 °C;
IR (KBr pellet, cm⁻¹): 3260, 3024, 2963, 1551, 1315, 1140;
¹H NMR(400MHz, CDCl₃): δ 7.26 (d, 2H, J =8.0Hz), 7.23 (s, 1H), 7.12 (d, 2H, J =8.0Hz), 6.98 (s, 1 H), 6.96 (dd, 1 H, J =17.6, 11.2Hz), 6.28 (s, 1H), 5.63 (d, 1 H, J =17.6Hz), 5.29 (d, 1 H, J =11.2Hz), 4.51 (d, 4H, J =12.0Hz), 2.90 (s, 3H), 2.27 (s, 3H), 1.21 (s, 9H)

### Example 6: N-(4-{1-[3-(4-t-butylbenzyl)thioureido]ethyl}-2-fluoro-6-vinyl-phenyl) methanesulfonamide

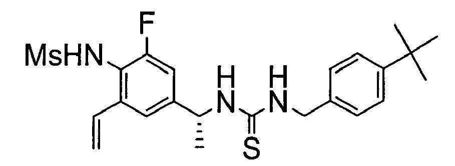

### Step 1: 1-(4-Amino-3-fluorophenyl)ethanone

A 25 ml of two neck round-bottom flask was filled with argon gas and the solution of 2-Fluoro-4-iodophenylamine (1500mg, 6.33mmol) in DMF, Palladium(Π) acetate (0.19mmol, 42.62mg), 1,3-bisdiphenyl phosphinopropane(0.06eq, 0.38mmol, 156.65mg), Thallium( l )acetate (6.96mmol, 1834.19mg), and Butyl vinyl ether(2eq, 12.66mmol, 1.64ml) were put into the flask. The reaction mixture was heated and stirred for 15hrs. The reaction mixture was poured into the THF solution, and then 10% HCl was added slowly. The reaction mixture was extracted with ethyl acetate(300×3), washed with H₂O, and brine. The combined organic solution was dried over Na₂SO₄ and then purified with column chromatography (n-Hx: EA= 3: 1) to yield a pale yellow solid (343.0mg, 35.40%).
Mp:77~79°C;
IR(KBr pellet, cm⁻¹): 3373, 3326, 1663, 1296;
¹H NMR(400MHz, CDCl₃): δ 7.53(?, 2H), 6.69(?, 1H), 3.41(s, 2H), 2.43(s, 3H).

### Step 2: 1-(4-Amino-3-fluoro-5-iodophenyl)ethanone

1-(4-Amino-3-fluorophenyl)ethanone (0.30mmol, 45.6mg) was added in the acetonitrile, and then NIS (0.33mol, 73.73mg) was added. A reaction mixture was stirred for 12 hrs. A reaction mixture was quenched by sodium thiosulfate. A reaction mixture was extracted with EtOAC and H₂O, a combined organic layer was washed with brine and dried over Na₂SO₄ and then concentrated in vacuo. The remain layer was purified with column chormactography (n-Hexane: EtOAc = 7 : 1) to yield a brownish solid (53.92mg, 64.43%).
Mp: 124~126 °C;
IR (KBr pellet): 3455, 3331, 3073, 2921, 1659, 1259 cm⁻¹;
¹H NMR(400MHz, CDCl₃) : δ 8.01 (dd, 1 H, J =1.6, 1.2Hz), 7.65(dd, 1H, *J* =11.6, 2.0Hz), 4.61(bs, 2H), 2.46(s, 3H).

### Step 3: 2-Methylpropane-2-sulfinic acid [1-(4-amino-3-fluoro-5-iodophenyl)ethyl]amide

1-(4-Amino-3-fluoro-5-iodophenyl)ethanone (0.36mmol, 100mg), Ti(OEt)₄ (0.59mmol, 122.68*µ*ℓ), (R)-(+)-2-methyl-2-propane sulfinamide(0.32mmol, 39.27mg) were added in THF solution. A reaction mixture was heated and stirred for 12 hrs. After confirming the completion of the reaction with TLC, a reaction mixture was cooled down to -40°C. NaBH₄ (1.19mmol, 45.08mg) was added into the reaction mixture. A reaction mixture was stirred for 12 hrs at -40°C. MeOH was added into the reaction mixture. The reaction mixture was heated to room temperature. A reaction mixture was filtered with Celite. The filterate was extracted with EtOAC, washed with H₂O and brine, dried over Na₂SO₄ and then concentrated in vacuo. The remain layer was purified with column chromatography (n-Hexane: EtOAc = 3 : 1) to yield a brownish syrup (29.1mg, 20.30%).
[α]_{D}²⁰ : -6.0 (CHCl₃, c 0.24);
IR (KBr pellet) : 3322, 3211, 2974, 1491, 1051, 717 cm⁻¹;
¹H NMR (400MHz, CDCl₃) : δ 7.34(s, 1 H), 6.96(dd, 1 H, *J*=11.2, 2.0Hz), 4.34(qd, 1H, *J*=6.4, 2.8Hz), 3.33(d, 1H, *J*=2.0Hz), 1.41(d, 3H, *J*=6.4Hz), 1.18(s, 9H).

### Step 4: 4-(1-Aminoethyl)-2-fluoro-6-iodophenylamine

2-methylpropane-2-sulfinic acid [1-(4-amino-3-fluoro-5-iodophenyl)ethyl]amide (0.07mmol, 29.1 mg) was added in 1 ml of MeOH. 4N HCl in 0.25mi of 1,4-dioxane was added. A reaction mixture was stirred for 12 hrs. A reaction mixture was concentrated in vacuo. A residue was filtered with glass filter. A filterate was concentrated in vacuo to yield a brownish solid (31.2mg).
Mp: 180~182 °C;
[α]D²⁰: +3.27 (CHCl₃, *c* 0.41);
IR (KBr pellet) : 3354, 3018, 2966, 1283, 756 cm⁻¹;
¹H NMR(400MHz, CDCl₃) : δ 7.72(s, 1H), 7.35(dd, 1 H, *J*=11.2, 2.0Hz), 4.41 (q, 1H, J=6.8Hz), 1.58(d, 3H, J=6.8Hz), 1.30(s, 3H).

### Step 5: [1-(4-Amino-3-fluoro-5-iodophenyl)ethyl]carbamic acid t-butylester

A 25 ml of two-necked flask was filled with argon gas and the 4-(1-aminoethyl)-2-fluoro-6-iodophenylamine (0.18mmol, 50.0mg) was dissolved in THF. BOC₂O (0.20mol, 45.18mg), DMAP (0.02mol, 2.20mg), and TEA (0.23mol, 32.61 mg) were added. A reaction mixture was stirred for 12 hrs. A reaction mixture was extracted with EtOAc, washed with H₂O and brine, dried over Na₂SO₄, and concentrated in vacuo. A residue was purified with column chromatography (n-Hexane: EtOAc = 7:1) to yield a solid (65.9mg, 94.44%).
Mp: 88~90 °C;
[a]_{D}²⁰: 33.35 (CHCl₃, c 2.98);
IR (KBr pellet) : 3364, 3026, 2958, 1696, 1169 cm⁻¹;
¹H NMR(400MHz, CDCl₃): δ 7.31(s, 1H), 6.90(d, 1H, J=11.6Hz), 4.72(s, 1H), 4.59(s, 1H), 3.76(bs, 2H), 1.38(s, 9H), 1.3(d, 3H, J=6.8Hz).

### Step 6: [1-(4-Amino-3-fluoro-5-vinylphenyl)ethyl]carbamic acid t-butylester

A 25 ml of two-necked flask was filled with argon gas and the Pd(PPh₃)₄ (0.01 mmol, 11.79mg) and LiCl (0.48mmol, 20.58mg) were added to DMF. [1-(4-Amino-3-fluoro-5-vinylphenyl)ethyl]carbamic acid *t-butyl* ester (0.17mmol, 65.9mg) and tributylvinyltin (0.25mmol, 74.52*µ*ℓ) were added. A reaction mixture was refluxed for 12 hrs. A reaction solvent was removed in vacuo. A reaction mixture was extracted with EtOAc, washed with H₂O and brine, dried over Na₂SO₄, and concentrated in vacuo. A residue was purified with column chromatography (n-Hexane: EtOAc = 7:1) to yield a yellow solid (23.4 mg, 47.06%).
Mp : 59~61°C;
[α]_{D}²⁰: +47.0 (CHCl₃, *c* 0.10);
IR(KBr pellet) : 3357, 3088, 2975, 1696, 1640, 1168 cm⁻¹;
¹H NMR(400MHz, CDCl₃) : δ 6.93(s, 1 H), 6.81 (dd, 1 H, J=11.6, 2.0Hz), 6.65(dd, 1H, *J*=17.2, 11.2Hz), 5.29(dd, 1H, *J*=11.2, 1.2Hz),4.64(s, 1H), 4.61(s, 1H), 3.66(bs, 2H), 1.35(s, 12H).

### Step 7: [1-(3-Fluoro-4-methanesulfonylamino-5-vinylphenyl)ethyl] carbamic acid t-butyl ester

A 25 ml of two-necked flask was filled with argon gas and the [1-(4-Amino-3-fluoro-5-vinylphenyl)ethyl]carbamic acid *t-butyl* ester (0.08mmol, 23.4mg) was added to methylene chloride. The reaction mixture was cooled down to 0°C. Methanesulfonyl chloride (0.40mmol, 32.32mmol) and TEA (0.24mmol, 33.45mg) were added into the reaction mixture. A reaction mixture was heated to room temperature. A reaction was quenched by adding NaHCO₃ solution. A reaction mixture was extracted with CH₂Cl₂. A combined organic layer was washed with CuSO₄, H₂O and brine, dried over Na₂SO₄, and concentrated in vacuo. A residue was added into the THF: H₂O = 2:1 soln. NaOH (0.40 mmol, 16 mg) was added. A reaction mixture was stirred for 12 hrs. After confirming the completion of the reaction with TLC, a reaction mixture was acidified with 10% HCl soln. A reaction mixture was stirred for 12 hrs. A reaction mixture was extracted with EtOAc, washed with H₂O and brine, dried over Na₂SO₄ , and concentrated in vacuo. A residue was purified with column chromatography (n-Hexane: EtOAc = 7:1) to yield a brownish syrup (20.2mg, 75.0%).
[α]_{D}²⁰ : +110.0 (CHCl₃, c 0.05);
IR(KBr pellet) : 3235, 2977, 1685, 1156 cm⁻¹;
¹H NMR(400MHz, CDCl₃) : δ 7.28(s, 1H), 7.10(dd, 1H, *J*=18.0, 11.2Hz), 6.95(d, 1H, *J*=10.4Hz), 6.16(s, 1H), 5.73(d, 1H, *J*=17.6Hz), 5.37(d, 1H, *J* =11.2Hz), 4.80(s, 1H), 4.69(s, 1 H), 2.99(s, 3H), 1.35(s, 12H).

### Step 8: N-[4-(1-Aminoethyl)-2-fluoro-6-vinylphenyl]methanesulfonamide

[1-(3-fluoro-4-methanesulfonylamino-5-vinyl phenyl)ethyl]carbamic acid *t-butyl* ester (0.06mmol, 20.2mg) was dissolved in methylene chloride, and 5~6 drops of CF₃COOH was added. A reaction mixture was stirred for 12 hrs. A toluene was added. A reaction mixture was concentrated in vacuo to yield a brownish syrup (20.8mg, 100.0%).
¹H NMR(400MHz, CD₃OD): δ 7.60(s, 1 H), 7.25 (dd, 1 H, *J*=10.4, 2.0Hz), 7.16 (dd, 1H, J=18.0, 11.2Hz), 5.89 (d, 1H, J=17.6Hz), 5.43(d, 1H, J=11.2Hz), 4.48 (q, 1H, J=6.8Hz), 3.02(s, 3H), 1.61(d, 3H, J=6.8Hz).

### Step 9: N-(4-{1-[3-(4-t-butylbenzyl) thioureido]ethyl}-2-fluoro-6-vinylphenyl)methanesulfonamide

*N*-[4-(1-Aminoethyl)-2-fluoro-6-vinylphenyl]methane sulfon amide (0.08mmol, 30.0 mg), 1-*t*-*butyl*-4-isothiocyanatomethylbenzene(1.1eq, 0.09mmol, 20.35mg), and TEA(3eq, 0.24mmol, 33.45mg) were added in methylene chloride under argon atmosphere. The reaction mixture was stirred for 12 hr. The reaction mixture was purified with column chromatography (n-Hx: EA= 3: 1) to yield a title product (37.5 mg, 100%) as a white solid.
Melting point: 113~115°C;
[*a*]_{D}²⁰ : -30.13 (CHCl₃, *c* 1.1);
IR (KBr pellet, cm⁻¹): 3356, 3252, 3023, 2963, 1578, 1151;
¹H NMR (400MHz, CDCl₃): δ 7.25(d, 2H, *J* =8.0Hz), 7.24(s, 1H), 7.05(dd, 1H, *J* =17.6, 11.2Hz), 7.04(d, 2H, *J* =8.0Hz), 6.89(d, 1H, *J* =9.6Hz), 6.33(s, 1H), 5.67(d, 1H, *J* =17.6Hz), 5.34(d, 1H, *J* =11.2Hz), 5.12(s, 1 H), 4.48(s, 2H), 2.96(s, 3H), 1.39(d, 3H, *J* =6.8Hz), 1.21(s, 9H).

### Experimental Example: Biological potency test

### 1. ⁴⁵ Ca influx test

### 1) Separation of spinal dorsal root ganglia (DRG) in newborn rats and primary culture thereof

Neonatal (2-3 day old or younger than 2-3 day old) SD rats were put in ice for 5 minutes to anesthetize and disinfected with 70% ethanol. DRG of all part of spinal cord were dissected (Wood et al., 1988, J. Neurosci. 8, pp3208-3220) and collected in DME/F12 medium to which 1.2g/l sodium bicarbonate and 50mg/l gentamycin were added. The DRG were incubated sequentially at 37°C for 30 min in 200 U/ml collagenase and 2.5mg/ml trypsin, separately. The ganglia were washed twice with DME/F12 medium supplemented with 10% horse serum, triturated through a fire-polished Pasteur pipette, filtered through Nitex 80 membrane to obtain single cell suspension and the suspension was washed once more. This was subjected to centrifugation, then resuspended in cell culture medium at certain level of cell density. As the cell culture medium, DME/F12 medium supplemented with 10% horse serum was diluted with identical medium conditioned by C6 glioma cells 2 days on a confluent monolayer (1:1), and NGF (Nerve Growth Factor) was added to adjust 200ng/ml as final concentration. After the cells were grown for 2 days in medium where cytosine arabinoside (Ara-C, 100 µM) was added to kill dividing nonneuronal cells, medium was changed to one without Ara-C. The resuspended cells were plated at a density of 1500-2000 neurons/well onto Terasaki plates previously coated with 10 µg/ml poly-D-ornithine.

### 2) ⁴⁵ Ca influx experiments

DRG nerve cells from the primary culture of 2 days were equilibrated by washing 4 times with HEPES (10mM, pH 7.4)-buffered Ca ²⁺, Mg²⁺-free HBSS (H-HBSS). The solution in each well was removed from the individual well. Medium containing the test compound plus capsaicin (final concentration 0.5 µM) and ⁴⁵Ca (final concentration 10 µCi/ml) in H-HBSS was added to each well and incubated at room temperature for 10 mins. Terasaki plates were washed five times with H-HBSS and dried at room temperature. To each well, 0.3% SDS (10 µl) was added to elute ⁴⁵Ca. After the addition of scintillation cocktail into each well, the amount of ⁴⁵Ca influx into neuron was measured by counting radioactivity. Antagonistic activities of test compounds against vanilloid receptor were calculated as percent of the inhibition of maximal response of capsaicin at a concentration of 0.5 µM. In summary, all examples of the present invention showed good to excellent IC50 values between 50 and 300 nM, with most of the compounds having IC50 values below 100 nM.

**[Table 1]**

| Results of Calcium Influx Test | |
|---|---|
| **Examples** | **Antagonist** |
| | **Calcium Uptake Test (IC₅₀, µM)** |
| **1** | 0.054 |
| **2** | 0.33 |
| **3** | 0.035 |
| **4** | 0.093 |
| **5** | 0.19 |
| **6** | 0.12 |

### 2. Analgesic activity test: Mouse writhing test by inducing with phenyl-p-quinone

Male ICR mice (mean body weight 25g) were maintained in a controlled lighting environment (12 h on/ 12 h off) for experiment. Animals received an intraperitoneal injection of 0.3ml of the chemical irritant phenyl-p-quinone (dissolved in saline containing 5% ethanol to be a dose of 4.5mg/kg) and 6 mins later, the number of abdominal constrictions was counted in the subsequent 6 mins period. Animals (10 animals/group) received 0.2ml of test compounds solution in vehicle of ethanol/Tween 80/saline (10/10/80) intraperitoneally 30 min before the injection of phenyl-p-quinone. A reduction in the number of writhes responding to the test drug compound relative to the number responding in saline control group was considered to be indicative of an analgesic effect. Analgesic effect was calculated by % inhibition equation (% inhibition=(C-T)/C x 100), wherein C and T represent the number of writhes in control and compound-treated group, respectively (Table 2).

**[Table 2]**

| Test result of analgesic activity for writhing by phenyl-p-quinone | | |
|---|---|---|
| **Example** | **Dose (mg/kg)** | **Analgesic effect (%Inhibition)** |
| 1 | 0.3 | 93% |
| 3 | 1 | 57% |

### Industrial Applicability

As explained above, the compound according to the present invention is useful to preventing and treating of pain, migraine, arthralgia, neuralgia, neuropathies, nerve injury, skin disorder, urinary bladder hypersensitiveness, irritable bowel syndrome, fecal urgency, a respiratory disorder, irritation of skin, eye or mucous membrane, stomach-duodenal ulcer, inflammatory diseases, ear disease, and heart disease etc.

More specifically, the compound according to the present invention is useful to preventing and treating of acute pain, chronic pain, neuropathic pain, post-operative pain, rheumatic arthrodynia, osteoarthritis pain, postherpetic neuralgia, diabetic neuropathy, HIV-related neuropathy, neurodegeneration, stroke, neurotic/allergic/inflammatory skin disease, psoriasis, pruritus, prurigo, asthma, chronic obstructive pulmonary disease, urinary incontinence, inflammatory bowel disease, hyperacusis, tinnitus, vestibular hypersensitiveness, and inotropic ischemia.

## Claims

1. A compound of formula (I), an isomer thereof, or a pharmaceutically acceptable salt thereof : 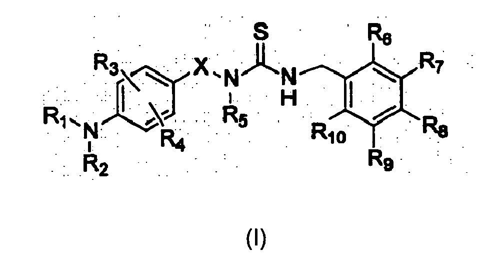 wherein,
X represents CHR₁₁ wherein R₁₁ represents hydrogen, halogen, or C1-C5 alkyl;
R₁ and R₂ independently represent hydrogen, -SO₂R₁₂, -SOR₁₂, C1-C5 alkyl, C1-C5 alkoxy, halo (C1-C5) alkyl, C2-C5 alkenyl, phenyl, phenyl (C1-C3) alkyl, or C1-C3 alkoxyphenyl, wherein R₁₂ represents hydrogen, C1-C5 alkyl, C2-C5 alkenyl, trifluoromethyl, phenyl, or benzyl;
R₃ represents C2-C5 alkenyl or C2-C5 alkynyl;
R₄ represents hydrogen, carboxy, C1-C5 alkyl, halogen, nitro, cyano, C2-C5 alkenyl, C2-C5 alkynyl, C1-C5 alkoxy, halo (C1-C5) alkyl, C1-C5 alkylcarbonyl, C1-C5 alkylcarbonylamino, C1-C5 alkylsulfonylamino, phenylsulfonylamino, or C1-C5 alkoxycarbonyl;
R₅ represents hydrogen, hydroxy, C1-C5 alkyl, or halo (C1-C5) alkyl; and
R₆, R₇, R₈, R₉ and R₁₀ independently represent hydrogen, C1-C5 alkyl, C2-C5 alkenyl, C2-C5 alkynyl, C1-C5 alkoxy, halo (C1-C5) alkyl, C1-C5 alkylthio, C1-C5 alkylsulfonyl, or halogen.

2. A compound according to claim 1, an isomer thereof, or a pharmaceutically acceptable salt thereof :
wherein,
X represents CHR₁₁ wherein R₁₁ represents hydrogen, halogen, or C1-C5 alkyl;
R₁ and R₂ independently represent hydrogen, -SO₂R₁₂, -SOR₁₂, C1-C5 alkyl, C1-C5 alkoxy, halo (C1-C5) alkyl, or C2-C5 alkenyl, wherein R₁₂ represents hydrogen, C1-C5 alkyl, C2-C5 alkenyl, or trifluoromethyl;
R₃ represents C2-C5 alkenyl or C2-C5 alkynyl;
R₄ represents hydrogen, C1-C5 alkyl, halogen, C2-C5 alkenyl, C2-C5 alkynyl, or halo (Cl-C5) alkyl;
R₅represents hydrogen, hydroxy, C1-C5 alkyl, or halo (C1-C5) alkyl; and
R₆, R₇, R₈, R₉ and R₁₀ independently represent hydrogen, C1-C5 alkyl, C2-C5 alkenyl, C2-C5 alkynyl, C1-C5 alkoxy, halo (C1-C5) alkyl, C1-C5 alkylthio, C1-C5 alkylsulfonyl, or halogen.

3. A compound according to claim 1 or 2, an isomer thereof, or a pharmaceutically acceptable salt thereof;
wherein,
R₃ represents ethenyl, ethynyl, propenyl, or propynyl; and
R₄ represents hydrogen, halogen, methyl, ethyl, propyl, isopropyl, butyl, isobutyl, ethenyl, ethynyl, or trifluoromethyl.

4. A compound according to anyone of the preceding claims, an isomer thereof, or a pharmaceutically acceptable salt thereof;
wherein,
R₁ represents hydrogen, and R₂ represents -SO₂R₁₂, wherein R₁₂ represents C1-C3 alkyl; and
R₈ represents isopropyl, trifluoromethyl or tert-butyl.

5. A compound according to anyone of the preceding claims, an isomer thereof, or a pharmaceutically acceptable salt thereof;
wherein,
X represents CHR₁₁ wherein R₁₁ represents hydrogen or C1-C5 alkyl;
R₁ represents hydrogen, and R₂ represents -SO₂R₁₂, wherein R₁₂ represents C1-C3 alkyl;
R₃ represents ethenyl or ethynyl;
R₄ represents hydrogen, halogen, methyl, ethyl, propyl, isopropyl, butyl, isobutyl, or trifluoromethyl;
R₅, R₆, R₇, R₉ and R₁₀ represent hydrogen; and
R₈ represents tert-butyl.

6. A compound of formula (1a), an isomer thereof, or a pharmaceutically acceptable salt thereof according to anyone of the preceding claims; 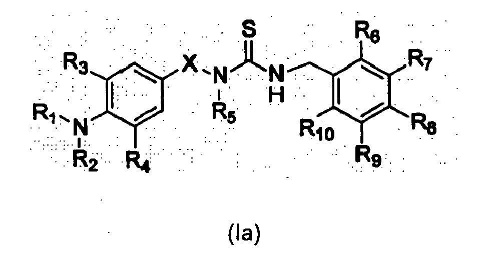 wherein,
R₁ represents hydrogen, and R₂ represents methanesulfonyl; and
R₃ and R₄ are both bounded to the phenyl ring in ortho-position to the methanesulfonylamino group.

7. A compound according to claim 1, an isomer thereof, or a pharmaceutically acceptable salt thereof, wherein the compound is selected from the group consisting of
N-{4-[3-(4-t-butylbenzyl)thioureido-methyl]-2-vinyl-phenyl}methanesulfonamide,
N-{4-[3-(4-t-butylbenzyl)thioureido-methyl]-2-trifluoromethyl-6-vinyl-phenyl}methanesulfonamide,
N-{4-[3-(4-t-butylbenzyl)thioureido-methyl]-2-fluoro-6-vinyl-phenyl}methanesulfonamide,
N-{4-[3-(4-t-butylbenzyl)thioureido-methyl]-2-chloro-6-vinylphenyl}methanesulfonamide,
N-{4-[3-(4-t-butylbenzyl)thioureido-methyl]-2-methyl-6-vinylphenyl}methanesulfonamide, and
(R)-N-(4-{1-[3-(4-t-butylbenzyl)thioureido]ethyl}-2-fluoro-6-vinyl-phenyl)methanesulfonamide.

8. A compound according to anyone of the preceding claims for use as a medicament.

9. A pharmaceutical composition comprising a compound, an isomer thereof, or a pharmaceutically acceptable salt thereof according to any one of claims 1 to 7, as an active ingredient, together with a pharmaceutically acceptable carrier.

10. A pharmaceutical composition for preventing and treating a condition associated with the pathological stimulation and/or aberrant expression of vanilloid receptors, wherein said composition comprise a compound, an isomer thereof, or a pharmaceutically acceptable salt thereof according to any one of claims 1 to 7; and a pharmaceutically acceptable carrier thereof.

11. The pharmaceutical composition for treating a condition selected from pain, inflammatory disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease (COPD), pruritus or prurigo comprising a compound, an isomer thereof or a pharmaceutically acceptable salt thereof according to any one of claims 1 to 7; and a pharmaceutically acceptable carrier.

12. The pharmaceutical composition according to claim 11 wherein the pain is or is associated with a condition selected from osteoarthritis, rheumatoid arthritis, ankylosing spondylitis, diabetic neuropathic pain, post-operative pain, non-inflammatory musculoskeletal pain (including fibromyalgia, myofascial pain syndrome and back pain), migraine and other types of headaches.

13. The pharmaceutical composition according to anyone of claims 9-12 **characterized in that** it is adapted for oral administration.

14. A method for inhibiting vanilloid ligand from binding to vanilloid receptor in a patient, comprising contacting cells expressing vanilloid receptor in the patient with a compound, an isomer thereof, or a pharmaceutically acceptable salt thereof according to any one of claims 1 to 7.

15. A method for preventing or treating a condition selected from pain, inflammatory disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease, pruritus or prurigo, which comprises administering to a mammal including a person in need thereof a therapeutically effective amount of a compound, an isomer thereof, or a pharmaceutically acceptable salt thereof according to any one of claims 1 to 7.

16. A method according to claim 15, wherein the pain is or is associated with a condition selected from osteoarthritis, rheumatoid arthritis, ankylosing spondylitis, diabetic neuropathic pain, post-operative pain, non-inflammatory musculoskeletal pain (including fibromyalgia, myofascial pain syndrome and back pain), migraine and other types of headaches.

17. Use of a compound, an isomer thereof, or a pharmaceutically acceptable salt thereof according to any one of claims 1 to 7 for prevention or treatment of a condition that is associated with the aberrant expression and/or aberrant activation of a vanilloid receptor.

18. Use of a compound, an isomer thereof, or a pharmaceutically acceptable salt thereof according to any one of claims 1 to 7, in preparation of a medicament for prevention or treatment of a condition that is selected from pain, inflammatory disease of the joints, urinary bladder hypersensitivity including urinary incontinence, stomach duodenal ulcer, irritable bowel syndrome (IBS) and inflammatory bowel disease (IBD), neurotic/allergic/inflammatory skin disease, psoriasis, asthma, chronic obstructive pulmonary disease, pruritus or prurigo.

19. Use of a compound according to claims 18, wherein the condition is pain or is associated with a condition selected from osteoarthritis, rheumatoid arthritis, ankylosing spondylitis, diabetic neuropathic pain, post-operative pain, non-inflammatory musculoskeletal pain (including fibromyalgia, myofascial pain syndrome and back pain), migraine and other types of headaches.
